# EUROPEAN PATENT SPECIFICATION

(11) **EP 2 377 517 B1**
(45) Date of publication and mention of the grant of the patent: **22.01.2020**
(21) Application number: 09835306.3
(22) Date of filing: 24.12.2009
(51) Int. Cl.: A61K 9/107, A61K 9/00, A61K 31/711, A61K 31/70, A61K 31/353, A61K 31/56, A61K 31/7105

(54) **PHARMACEUTICAL COMPOSITION CONTAINING AN ANIONIC DRUG, AND A PRODUCTION METHOD THEREFOR**
PHARMAZEUTISCHE ZUSAMMENSETZUNG AUS EINEM ANIONISCHEN ARZNEIMITTEL UND HERSTELLUNGSVERFAHREN DAFÜR
COMPOSITION PHARMACEUTIQUE CONTENANT UN MÉDICAMENT ANIONIQUE ET PROCÉDÉ DE PRÉPARATION ASSOCIÉ

(30) Priority: 26.12.2008 KR 20080134459; 24.12.2009 KR 20090130794
(43) Date of publication of application: 19.10.2011
(73) Proprietor: Samyang Biopharmaceuticals Corporation, Seoul 110-725 (KR)
(72) Inventor: KIM, Se-Ho, Daejeon 302-280 (KR); SON, Ji-Yeon, Gimhae-si Gyeongsangnam-do 621-775 (KR); LA, Muhn-Ho, Daejeon 302-170 (KR); CHOI, Sung-Won, Daejeon 302-120 (KR); SEO, Min-Hyo, Daejeon 302-782 (KR)
(74) Representative: Brann AB
(86) International application number: PCT/KR2009/007804
(87) International publication number: WO 2010/074540

(56) References cited:
- WO-A1-2005/107813
- WO-A1-2010/026537
- WO-A2-2004/026453
- WO-A2-2009/137689
- KR-A- 20060 130 057
- US-A1- 2005 013 812

## Description

### Technical Field of the Invention

This disclosure relates to an anionic drug-containing pharmaceutical composition comprising: an anionic drug as an active ingredient; a cationic lipid; and an amphiphilic block copolymer, wherein the anionic drug forms a complex with the cationic lipid, and the complex is entrapped in the micelle structure of the amphiphilic block copolymer, and a method of preparing the same.

### Background of the Invention

Safe and efficient drug delivery technologies have been studied for a long time in the treatment using anionic drugs, particularly nucleic acid material, and various delivery systems and delivery technologies have been developed. Particularly, delivery technologies using a viral delivery system using adenovius or retrovirus, etc., and a non-viral delivery system using cationic lipids, cationic polymers, etc. have been developed.

However, a technology using a viral delivery system is exposed to a risk such as non-specific immune reaction, etc., and it is known to have a lot of problems in commercialization due to the complex production process. Therefore, recent studies are progressed toward a non-viral delivery system using cationic lipids or cationic polymers to improve the disadvantages. Although the non-viral delivery system has inferior efficiency to the viral delivery system, it has less side effects and the production cost is inexpensive compared with viral delivery system.

Many studies have been conducted on non-viral delivery system used for delivery of nucleic acid material, and most representative examples thereof include a complex of cationic lipid and nucleic acid (lipoplex) and a complex of a polycationic polymer and nucleic acid (polyplex). Many studies on the cationic lipid or polycationic polymer have been progressed because it stabilizes anionic drugs by forming a complex by electrostatic interactions with the anionic drug and facilitates intracellular delivery (De Paula D, Bentley MV, Mahato RI, Hydrophobization and bioconjugation for enhanced siRNA delivery and targeting, RNA 13 (2007) 431-56; Gary DJ, Puri N, Won YY, Polymer-based siRNA delivery: Perspectives on the fundamental and phenomenological distinctions from polymer-based DNA delivery, J Control release 121 (2007) 64-73).

However, if cationic lipids or polycationic polymers studied so far are used in an amount required to obtain sufficient effects, serious toxicity, although less than viral delivery system, may be caused and thus it may be improper for the therapeutic use. And, although a lipid-nucleic acid complex which forms a complex compound through a bond between a cationic lipid and nucleic acid is widely used in a cell line experiment, it does not form a structure that can be stable in blood, and thus it cannot be used in the living body (see US 6,458,382).

A nucleic acid-cationic liposome complex or a cationic liposome comprising nucleic acid, which is one of the non-viral delivery system commonly used to deliver nucleic acid into the cells in the living body, consists of an amphiphilic lipid, a neutral lipid and a fusogenic lipid, etc., and nucleic acid material is attached to the outside of the liposome by electrostatic bond or captured inside (US2003-0073640, WO05/007196, US2006-0240093). However, the liposome delivery system may be easily captured by reticuloendothelial system (RES) and exhibit side effects with significant toxicity, and thus, it may not be appropriate for systemic application. And, another non-viral delivery system commonly used includes a cationic polymer, and a polycationic polymer including multivalent cationic charge per a polymer is predominantly used therefore. Particularly, commonly used polymer is polycationic polyethyleneimine (PEI), and the polycationic polymer binds with nucleic acid material by electrostatic interaction to form a nucleic acid-polymer complex thereby forming a nanoparticle. However, the polycationic polymer such as polyethyleneimine promotes apoptosis, and it is known that cytotoxicity increases as the molecular weight and the degree of branching of the polymer increase. Although polycationic polymers with low molecular weight are known to have low cytotoxicity, they cannot form an effective complex due to low charge density of the polymer, and thus, they cannot show the sufficient intracellular delivery and the sufficient stability in blood.

Therefore, it is required to develop an anionic drug delivery technology using the minimal amount of cationic polymer or cationic lipid to decrease toxicity, which is stable in blood and body fluid, and enables intracellular delivery to obtain sufficient effects. The delivery system using the nucleic acid material directly conjugated with a lipid or a polymer is being studied, but if a lipid or a polymer is directly conjugated with nucleic acid material, there are difficulties in terms of conjugation efficiency or quality control.

Meanwhile, there have been various attempts to use amphiphilic block copolymer as a drug delivery system that can solubilize a poorly water-soluble drug by forming a polymeric micelle and stabilize a poorly water-soluble drug in an aqueous solution (Korean Registered Patent No. 0180334). However, since the amphiphilic block copolymer cannot enclose hydrophilic drug such as nucleic acid in the polymeric micelle, it is not suitable for delivery of anionic drug including nucleic acid.

Meanwhile, many diseases result from the overexpression of disease genes or the expression of mutated genes. Since siRNA (short interfering RNA) inhibits the expression of specific genes in a sequence specific manner, it is highlighted as a therapeutic nucleotide drug. Particularly, siRNA is expected to overcome the problems of the antisense nucleotide or ribozyme because siRNA has more potency and more accurate gene selectivity compared with the antisense nucleotide or ribozyme. The siRNA is a short double-stranded RNA molecule and the number of nucleotides in each strand ranges from 15 to 30, and it inhibits the expression of corresponding gene by cleaving mRNA of gene with a sequence complementary thereto (McManus and Sharp, Nature Rev. Genet. 3:737 (2002); Elbashir, et al., Genes Dev. 15:188 (2001).

However, despite these advantages, siRNA is known to be rapidly degraded by nuclease in blood and rapidly excreted from the body through a kidney. It is also known that siRNA cannot easily pass a cell membrane because it is strongly negatively charged. Therefore, to use siRNA as a therapeutic agent, it is required to develop a delivery system that may stabilize siRNA in blood, may efficiently deliver it into target cells, and does not show toxicity.

WO2004/026453 discloses a particle comprising an active agent such as DNA or RNA, an amphiphilic binding molecule such as a cationic lipid, and an encapsulation material such as a hydrophobic polymer.

WO2009/137689 discloses an active agent delivery device comprising nucleic acid complexes comprising nucleic acid and a cationic carrier agent and a polymeric excipient that undergoes phase separation.

### Detailed Description of the Invention

### Technical Problem

The scope of this invention is defined by the claims. Any references in the description to methods of treatment refer to the compounds, pharmaceutical compositions and medicaments of the present invention for use in a method for treatment of the human (or animal) body by therapy.

Accordingly, one aspect of the present invention provides a pharmaceutical composition capable of effectively delivering anionic drugs in the body.

Another aspect of the present invention provides a method of preparing the pharmaceutical composition capable of effectively delivering anionic drugs in the body.

### Technical Solution

The pharmaceutical composition according to the present invention is consisting of an anionic drug as an active ingredient;
a cationic lipid; and
an amphiphilic block copolymer, optionally at least one fusogenic lipid, and
an aqueous solution or an assistant agent for freeze drying, the anionic drug forms a complex with the cationic lipid, and the complex is entrapped in the core-shell type polymeric micelle structure of the amphiphilic block copolymer of an A-B type di-block copolymer comprising of a hydrophilic A block forming a shell and having a number avreage molecular weight of 200 to 50,000 Dalton and a hydrophobic B block forming a core and having a number average molecular weight of 50 to 50,000 Dalton in an aqueous solution, and wherein the content of the amphiphilic block copolymer is 40-99.98 wt% based on the total dry weight of the composition; and tocopherol, cholesterol, or C10-C24 fatty acid is optionally chemically conjugated to a hydroxyl group of the hydrophobic block end, wherein the hydrophilic A block is polyalkyleneglycol, and the hydrophobic B block is one or more selected from the group consisting of polyester, polyanhydride, polyamino acid, polyorthoester, and polyphosphazine. The composition may be used for delivery of the anionic drug contained as the active ingredient.

Another embodiment provides use of the composition comprising an anionic drug as an active ingredient; a cationic lipid; and an amphiphilic block copolymer, wherein the anionic drug forms a complex with the cationic lipid, and the complex is entrapped in the micelle structure of the amphiphilic block copolymer, for delivery of an anionic drug.

Yet another embodiment provides a method of delivering an anionic drug comprising the administration of the composition defined here above.

The anionic drug forms a complex with the cationic lipid, and the complex is entrapped in the micelle structure of the amphiphilic block copolymer, to a patient in need thereof. The patient may include mammals, preferably human, primates, rodents, and the like.

And, a method of preparing the composition according to the present invention may comprise:
(a) dissolving the anionic drug and the cationic lipid in a water-miscible organic solvent or a mixed solvent of an aqueous solution and an organic solvent so as to separate the phases;
(b) separating the organic solvent layer of (a);
(c) mixing the organic solvent of (b) with the amphiphilic block copolymer and removing the organic solvent; and
(d) adding an aqueous solution to the mixture from which the organic solvent is removed so as to form a micelle

According to another embodiment, a method of preparing the composition according to the present invention may comprise:
(a) dissolving the anionic drug, the cationic lipid and the amphiphilic block copolymer in a water-miscible organic solvent or a mixed solvent of an aqueous solution and an organic solvent;
(b) removing the organic solvent layer of (a); and
(c) adding an aqueous solution to the mixture of (b) from which the organic solvent is removed so as to form a micelle.

Hereinafter, the present invention will be explained in detail.

According to one embodiment, the anionic drug and the cationic lipid are entrapped in the micelle structure of the amphiphilic block copolymer while forming a complex of the anionic drug and the lipid by electrostatic interactions.

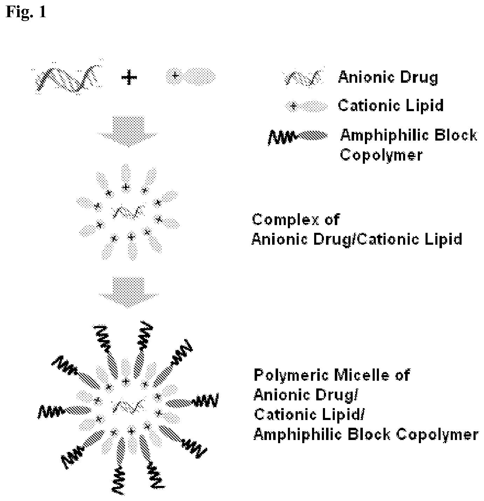

Fig. 1 schematically shows the structure of the polymeric micelle delivery system according to one embodiment of the present invention in which the anionic drug and the cationic lipid are enclosed. As shown in Fig. 1, the anionic drug binds to the cationic lipid by electrostatic interactions, so as to form a complex of the anionic drug and the cationic lipid, and the formed complex of the anionic drug and the cationic lipid is entrapped in the micelle structure of the amphiphilic block copolymer.

When the complex of the anionic drug and the cationic lipid is entrapped in the micelle structure of the amphiphilic block copolymer, it may have improved stability in blood or in a body fluid. According to one embodiment, the particle size of the micelle may be 10 to 200 nm, specifically 10 to 150 nm. The particle size is determined considering the stability of the micelle structure, the contents of the constitutional ingredients, absorption of anionic drugs in the body, and convenience of sterilization as a pharmaceutical composition.

The anionic drug may include any material that is negatively charged in an aqueous solution and has pharmacological activity. According to one embodiment, the anionic property may be provided from at least one functional group selected from the group consisting of carboxylic group, phosphate group, and sulfate group. According to one embodiment, the anionic drug may be a multi-anionic drug or nucleic acid.

The nucleic acid may be a nucleic acid drug such as polynucleotide derivatives wherein deoxyribonucleic acid, ribonucleic acid or backbone, sugar or base is chemically modified or the end is modified, and more specific examples may include RNA, DNA, siRNA (short interfering RNA), aptamer, antisense ODN (oligodeoxynucleotide), antisense RNA, ribozyme, DNAzyme, and a combination thereof. And, the backbone, sugar or base of the nucleic acid may be modified or the end may be modified for the purpose of increasing blood stability or weakening immune reactions, and the like. Specifically, a part of phosphodiester bond of nucleic acid may be substituted by phosphorothioate or boranophosphate bond, or at least one kind of nucleotide wherein various functional groups such as methyl group, methoxyethyl group, fluorine, and the like are introduced in 2'-OH positions of a part of ribose bases may be included.

According to another embodiment, the end of the nucleic acid may be modified by at least one selected from the group consisting of cholesterol, tocopherol, and C10-C24 fatty acid. For example, for siRNA, 5'end or 3'end, or both ends of sense and/or antisense strand may be modified, and preferably, the end of sense strand may be modified.

The cholesterol, tocopherol and fatty acid may include analogues, derivatives and metabolites thereof.

The siRNA refers to duplex RNA or single strand RNA having a double stranded form in the single strand RNA, which may reduce or inhibit the expression of a target gene by mediating degradation of mRNA complementary to the sequence of siRNA if siRNA exists in the same cell as the target gene does. The bond between the double strands is made by hydrogen bond between nucleotides, not all nucleotides in the double strands should be complementarily bound with the corresponding nucleotides, and both strands may be separated or may not be separated. According to one embodiment, the length of the siRNA may be about 15∼60 nucleotides (it means the number of nucleotides of one of double stranded RNA, i.e., the number of base pairs, and in the case of a single stranded RNA, it means the length of double strands in the single stranded RNA), specifically about 15∼30 nucleotides, and more specifically about 19∼25 nucleotides.

According to one embodiment, the double stranded siRNA may have overhang of 1-5 nucleotides at 3' or 5' end or both ends. According to another embodiment, it may be blunt without overhang at both ends. Specifically, it may be siRNA disclosed in US20020086356 and US7056704.

According to one embodiment, siRNA may have a symmetrical structure with the same lengths of two strands, or it may have a non-symmetrical structure with one strand shorter than the other strand. Specifically, it may be a non-symmetrical siRNA (small interfering RNA) molecule of double strands consisting of 19-21 nucleotide (nt) antisense; and 15∼19nt sense having a sequence complementary to the antisense, wherein 5'end of the antisense is blunt end, and the 3'end of the antisense has 1-5 nucleotide overhang. Specifically, it may be siRNA disclosed in WO09/078685.

The anionic drug of the present invention may be included in the content of 0.001 to 10 wt%, specifically 0.01 to 5 wt%, based on the total weight of the composition. If the content is less than 0.001 wt%, the amount of delivery system is too large compared to the drug, and thus, side effect may be caused by delivery system, and if it exceeds 10 wt%, the size of micelle may be too large to decrease stability of the micelle and increase loss rate during filter sterilization.

According to one embodiment, the cationic lipid forms a complex with the anionic drug by electrostatic interactions, and the complex is entrapped in the micelle structure of the amphiphilic block copolymer. The cationic lipid may include any lipid capable of forming a complex with the anionic drug by electrostatic interactions, and specific example thereof may include N,N-dioleyl-N,N-dimethylammoniumchloride (DODAC), N,N-distearyl-N,N-dimethylammoniumbromide (DDAB), N-(1-(2,3-dioleoyloxy)propyl-N,N,N-trimethylammoniumchloride (DOTAP), N,N-dimethyl-(2,3-dioleoyloxy)propylamine (DODMA), 1,2-diacyl-3-trimethylammonium-propane (TAP), 1,2-diacyl-3-dimethylammonium-propane (DAP), 3β-[N-(N',N',N'-trimethylaminoethane)carbamoyl]cholesterol (TC-cholesterol), 3 β[N-(N',N'-dimethylaminoethane)carbamoyl]cholesterol (DC-cholesterol), 3β[N-(N'-monomethylaminoethane)carbamoyl]cholesterol (MC-cholesterol), 3β[N-(aminoethane)carbamoyl]cholesterol (AC-cholesterol), cholesteryloxypropane-1-amine (COPA), N-(N'-aminoethane)carbamoylpropanoic tocopherol (AC-tocopherol), N-(N'-methylaminoethane)carbamoylpropanoic tocopherol (MC-tocopherol), and a combination thereof. Specifically, to decrease toxicity induced by cationic lipid, it may be preferable to use less polycationic lipid having high charge density, and more specifically, one functional group capable of exhibiting positive charge in the molecule in an aqueous solution may be included. Specific example of the cationic lipid may include 3β-[N-(N',N',N' -trimethylaminoethane)carbamoyl] cho lesterol (TC-cholesterol), 3 β[N-(N',N' -dimethylaminoethane)carbamoyl]cholesterol (DC-cholesterol), 3β[N-(N'-monomethylaminoethane)carbamoyl]cholesterol (MC-cholesterol), 3 β[N-(aminoethane)carbamoyl] cho lesterol (AC-cholesterol), N-(1-(2,3-dioleoyloxy)propyl-N,N,N-trimethylammoniumchloride (DOTAP), N,N-dimethyl-(2,3-dioleoyloxy)propylamine (DODMA), N,N,N-trimethyl-(2,3-dioleoyloxy)propylamine (DOTMA), and a combination thereof.

The cationic lipid may be included in the content of 0.01 to 50 wt%, specifically 0.1 to 10 wt%, based on the total weight of the composition. If the content is less than 0.01 wt%, it may not be sufficient to form a complex with the anionic drug, and if it exceeds 50 wt%, the size of micelle may be too large to decrease stability of the micelle and increase loss rate during filter sterilization.

The cationic lipid binds with the anionic drug by electrostatic interactions so as to form a complex with the anionic drug. According to one embodiment, the ratio of quantity of electric charge of the cationic lipid (N) and the anionic drug (P) (N/P: the ratio of the positive electric charge of the cationic lipid to the negative electric charge of the anionic drug) is 0.1 to 128, specifically 0.5 to 32, more specifically 1 to 16. If the ratio (N/P) is less than 0.1, it may be difficult to form a complex including a sufficient amount of anionic drug. On the other hand, if the ratio (N/P) exceeds 128, toxicity may be induced.

According to the invention, the amphiphilic block copolymer is an A-B type block copolymer including a hydrophilic A block and a hydrophobic B block. The A-B type block copolymer forms a core-shell type polymeric micelle in an aqueous solution, wherein the hydrophobic B block forms a core and the hydrophilic A block forms a shell.

According to the invention, the hydrophilic A block is polyalkyleneglycol. More specifically, the hydrophilic A block may be at least one selected from the group consisting of monomethoxy polyethylene glycol, monoacetoxy polyethylene glycol, polyethylene glycol, and a copolymer of polyethylene and propylene glycol. The hydrophilic A block has a number average molecular weight of 200 to 50,000 Dalton, specifically 1,000 to 20,000 Dalton, more specifically 1,000 to 5,000 Dalton.

And, if necessary, a functional group or a ligand that may bind to a specific tissue or cell, or a functional group capable of promoting intracellular delivery may be chemically conjugated to the end of the hydrophilic A block so as to control the distribution of the polymeric micelle delivery system in the body or increase the efficiency of the intracellular delivery of polymeric micelle delivery system. The functional group or ligand may include monosaccharide, polysaccharide, vitamins, peptides, proteins, an antibody to a cell surface receptor, and a combination thereof. More specific examples thereof may include anisamide, vitamin B9 (folic acid), vitamin B12, vitamin A, galatose, lactose, mannose, hyaluronic acid, RGD peptide, NGR peptide, transferrin, an antibody to a transferring receptor, and a combination thereof.

The hydrophobic B block is a polymer having excellent biocompatibility and biodegradability, and is selected from the group consisting of polyester, polyanhydride, polyamino acid, polyorthoester, and polyphosphazine. More specific examples thereof may include polylactide, polyglycolide, polycaprolactone, polydioxane-2-one, a copolymer of polylactide and glycolide, a copolymer of polylactide and polydioxane-2-one, a copolymer of polylactide nad polycaprolactone, a copolymer of polyglycolide and polycaprolactone, and a combination thereof. The hydrophobic B block has a number average molecular weight of 50 to 50,000 Dalton, specifically 200 to 20,000 Dalton, more specifically 1,000 to 5,000 Dalton. And, to increase hydrophobicity of the hydrophobic block for improving the stability of the micelle, tocopherol, cholesterol, or C10-C24 fatty acid may be chemically conjugated to a hydroxyl group of the hydrophobic block end.

The amphiphilic block copolymer comprising the hydrophilic block (A) and the hydrophobic block (B) is included in the content of 40 to 99.98 wt%, specifically 85 to 99.8 wt%, more specifically 90 to 99.8 wt%, based on the total dry weight of the composition. If the content of the amphiphilic block copolymer is less than 40 wt%, the size of the micelle may become so large that the stability of the micelle may be decreased and the loss during filter sterilization may be increased, and if it exceeds 99.98wt%, the content of anionic drug that can be incorporated may become too small.

According to another embodiment, the amphiphilic block copolymer may include 40 to 70 wt% of the hydrophilic block (A), specifically 50 to 60 wt% of the hydrophilic block (A), based on the weight of the copolymer. If the ratio of the hydrophilic block (A) is less than 40 wt%, solubility of the polymer in water is low, and thus it may be difficult to form a micelle. On the other hand, if it exceeds 70 wt%, hydrophilicity may be too high and thus stability of the polymeric micelle is low, and it may be difficult to solubilize a complex of the anionic drug and the cationic lipid.

According to one embodiment, the amphiphilic block copolymer allows enclosure of the complex of the anionic drug and the cationic lipid in the micelle structure in an aqueous solution, wherein the ratio of the weight of the complex of the anionic drug and the cationic lipid (a) to the weight of the amphiphilic block copolymer (b) [a/b X 100; (the weight of the anionic drug + the weight of the cationic lipid)/the weight of the amphiphilic block copolymer X 100] may be 0.001 to 100 wt%, specifically 0.01 to 50 wt%, more specifically 0.1 to 10%. If the weight ratio is less than 0.001 wt%, the content of the complex of the anionic drug and the cationic lipid may become too low, and thus it may be difficult to satisfy effective content of the anionic drug, and if it exceeds 100 wt%, a micelle structure of appropriate size may not be formed considering the molecular weight of the amphiphilic block copolymer and the amount of the complex of the anionic drug and the lipid.

According to one embodiment, the pharmaceutical composition of the present invention may further comprise a fusogenic lipid in the content of 0.01 to 50 wt%, specifically 0.1 to 10 wt%, based on the total weight of the composition, in order to increase intracellular delivery efficiency of the anionic drug.

The fusogenic lipid form a complex with the anionic drug, the cationic lipid by the hydrophobic interactions while mixing the anionic drug with the cationic lipid, and the complex comprising the fusogenic lipid is entrapped in the micelle structure of the amphiphilic block copolymer. According to one embodiment, the fusogenic lipid may be phospholipid, cholesterol, tocopherol, or a combination thereof.

Specifically, the phospholipid may be selected from phosphatidylethanolamin (PE), phosphatidylcholine (PC), phosphatidic acid, or a combination thereof. The phosphatidylethanolamin (PE), phosphatidylcholine (PC) and the phosphatidic acid may be bound to one or two C10-24 fatty acid. The cholesterol and tocopherol may include analogues, derivative, and metabolites thereof.

Specifically, the fusogenic lipid may include dilauroyl phosphatidylethanolamine, dimyristoyl phosphatidylethanolamine, dipalmitoyl phosphatidylethanolamine, distearoyl phosphatidylethanolamine, dioleoyl phosphatidylethanolamine, dilinoleoyl phosphatidylethanolamine, 1-palmitoyl-2-oleoyl phosphatidylethanolamine, 1,2-diphytanoyl-3-sn-phosphatidylethanolamine, dilauroyl phosphatidylcholine, dimyristoyl phosphatidylcholine, dipalmitoyl phosphatidylcholine, distearoyl phosphatidylcholine, dioleoyl phosphatidylcholine, dilinoleoyl phosphatidylcholine, 1-palmitoyl-2-oleoyl phosphatidylcholine, 1,2-diphytanoyl-3-sn-phosphatidylcholine, dilauroyl phosphatidic acid, dimyristoyl phosphatidic acid, dipalmitoyl phosphatidic acid, distearoyl phosphatidic acid, dioleoyl phosphatidic acid, dilinoleoyl phosphatidic acid, 1-palmitoyl-2-oleoyl phosphatidic acid, 1,2-diphytanoyl-3-sn-phosphatidic acid, cholesterol, tocopherol, and a combination thereof.

According to preferred embodiment, the fusogenic lipid may include dioleoyl phosphatidylethanolamine (DOPE), 1,2-dipalmitoleoyl-sn-glycero-3-phosphocholine (DPPC), 1,2-dioleoyl-sn-glycero-3-phosphocholine (DOPC), 1,2-dipalmitoleoyl-sn-glycero-3-phosphoethanolamine (DPPE), and a combination thereof.

The present invention also provides a method of preparing the in claim 1 defined pharmaceutical composition comprising an amphiphilic diblock copolymer micelle containing anionic drug.

According to one embodiment, the method of preparing a composition comprising an anionic drug, a cationic lipid, and an amphiphilic block copolymer comprises:
(a) dissolving the anionic drug and the cationic lipid in a water-miscible organic solvent or a mixed solvent of an aqueous solution and an organic solvent so as to separate the phases;
(b) separating the organic solvent layer of (a);
(c) mixing the organic solvent of (b) with the amphiphilic block copolymer and removing the organic solvent; and
(d) adding an aqueous solution to the mixture from which the organic solvent is removed so as to form a micelle.

In the step (a), the anionic drug and the cationic lipid are mixed in a water-miscible organic solvent, or a mixed solvent of an aqueous solution and an organic solvent to form a complex. Specifically, the water-miscible organic solvent may include acetone, ethanol, methanol, acetic acid, and a combination thereof, and the organic solvent of the mixed solvent may include ethyl acetate, acetonitrile, methylene chloride, chloroform, dioxane, and a combination thereof. The aqueous solution may include distillated water, water for injection, and a buffer solution. The amount of the complex of the anionic drug and the cationic lipid dissolved in the solvent may be 0.1 ∼ 100 wt%, specifically 0.1∼10 wt%, more specifically 0.1 ∼ 1 wt%, based on the amount of the used solvent. If the amount is 100 wt% or more, yield may be rapidly decreased when the complex of the anionic drug and the cationic lipid is extracted with an organic solvent in the step (b) below.

In the step (b), the complex of the anionic drug and the cationic lipid is recovered by phase separation. An aqueous solution and an organic solvent may be added to the solvent of the step (a) to induce phase separation. And, to shorten the phase separation time, a centrifugation process may be added.

In the step (c), an amphiphilic block copolymer is added to the extracted organic solvent and mixed, and then, the organic solvent is removed by evaporation.

In the step (d), the complex of the anionic drug and the cationic lipid is entrapped in the micelle structure of the amphiphilic block copolymer by dissolving the remaining mixture with an aqueous solution. The aqueous solution may be distillated water, water for injection, or a buffer solution, and the amount may be such that the concentration of the amphiphilic block copolymer may become 10 to 300 mg/mL. If the concentration of the amphiphilic block copolymer is less than 10 mg/mL, the volume of the aqueous solution may become too large thus rendering it difficult to handle during the preparation process, and if it exceeds 300 mg/mL, the viscosity of the aqueous solution may be too high thus rendering it difficult to prepare a micelle.

According to yet another embodiment, a method of preparing the in claim 1 defined composition for delivery of an anionic drug comprising an anionic drug, a cationic lipid, and an amphiphilic block copolymer comprises:
(a') dissolving the anionic drug, the cationic lipid and the amphiphilic block copolymer in a water-miscible organic solvent or a mixed solvent of an aqueous solution and an organic solvent;
(b') removing the organic solvent of (a'); and
(c') adding an aqueous solution to the mixture of (b') so as to form a micelle.

In the step (a'), the anionic drug, the cationic lipid, and the amphiphilic block copolymer are mixed in a water-miscible organic solvent, or a mixed solvent of an aqueous solution and an organic solvent to form a complex. Specifically, the water-miscible organic solvent may include acetone, ethanol, methanol, acetic acid, and a combination thereof, and the organic solvent of the mixed solvent may include ethyl acetate, acetonitrile, methylene chloride, chloroform, dioxane, and a combination thereof. The aqueous solution may include distillated water, water for injection, and a buffer solution.

In the step (b'), the organic solvent is removed by evaporation.

In the step (c'), the remaining mixture is dissolved in an aqueous solution, thereby enclosing the complex of the complex of the anionic drug and the cationic lipid in the micelle structure of the amphiphilic block copolymer. The kind and the amount of the aqueous solution are as described above.

According to yet another embodiment, for a composition comprising a fusogenic lipid, the fusogenic lipid may be added together when adding the amphiphilic block copolymer for forming a micelle, and for example, it may be added in the step (c) or (a').

According to yet another embodiment, the method may further comprise (e) adding assistant material for freeze drying, after the step (d) of (c').

According to one embodiment, the method may further comprise sterilizing the polymeric micelle aqueous solution obtained in the step (d) or (c') with a sterilization filter, before the (e) freeze drying.

According to one embodiment, the assistant material for freeze drying may include lactose, mannitol, sorbitol, sucrose, and a combination thereof. The assistant material for freeze drying is added to allow the freeze dried composition to maintain a cake form. According to another embodiment, the content of the assistant material for freeze drying may be 1 to 90 wt%, specifically 10 to 60 wt%, based on the total dry weight of the composition.

According to one embodiment, the amphiphilic block copolymer micelle composition containing an anionic drug may be prepared in the form of an aqueous solution, powder or a tablet. According to another embodiment, the composition may be prepared for injection. For example, the freeze dried composition may be reconstituted with distillated water for injection, a 0.9% saline solution, a 5% dextrose aqueous solution, and the like.

The micelle formed according to the preparation method of the present invention is stable in blood, and has the particle size of 10 to 200 nm, specifically 10 to 150 nm.

The pharmaceutical composition containing an anionic drug of the present invention may be administered in the route of blood vessel, muscle, subcutaneous, oral, bone, transdermal or local tissue, and the like, and it may be formulated in various forms such as a solution, a suspension for injection, a tablet, or a capsule, and the like.

The pharmaceutical composition containing an anionic drug of the present invention may increase stability of the anionic drug in blood or in body fluid by isolating the anionic drug from outside using the cationic lipid and the amphiphilic block polymer. And, the composition of the present invention may effectively deliver the anionic drug in the cell. And, the amphiphilic polymer has excellent biodegradability and biocompatibility.

### Brief Description of the Drawings

Fig 1. is a schematic diagram of the pharmaceutical composition containing an anionic drug according to one embodiment of the present invention.
Fig. 2 is an NMR measurement result of AC-tocopherol prepared by the preparation method according to one embodiment of the present invention.
Fig. 3 is an NMR measurement result of MC-tocopherol prepared by the preparation method according to one embodiment of the present invention.
Fig. 4 is an NMR measurement result of mPEG-PLA block copolymer polymerized by the preparation method according to Example 3 of the present invention.
Fig. 5 is an NMR measurement result of mPEG-PLA block copolymer polymerized by the preparation method according to Example 4 of the present invention.
Fig. 6 is an NMR measurement result of mPEG-PLA-tocopherol polymerized by the preparation method according to Example 5 of the present invention.
Fig. 7 is an NMR measurement result of mPEG-PLA-tocopherol polymerized by the preparation method according to Example 6 of the present invention.
Fig. 8 is an NMR measurement result of anisamide-PEG-PLA polymerized by the preparation method according to one embodiment of the present invention.

### Examples

Hereinafter, the present invention will be explained in detail with reference to the following Examples, however theses Examples are only to illustrate the invention and the scope of the invention is not limited thereto in any manner.

### [Example 1] Synthesis of

### AC-cholesterol(3β[N-(aminoethane)carbamoyl] cholesterol)

To synthesize AC-cholesterol, cholesteryl chloroformate (Sigma-Aldrich) and ethylenediamine (Sigma-Aldrich) were reacted as follows.

1g (2.23mmol) of cholesteryl chloroformate was dissolved in 20ml of chloroform, 20 equivalents of ethylenediamine was diluted with 30ml of chloroform in a separate reaction vessel, and the temperature was maintained at 4°C. The cholesteryl chloroformate solution was slowly introduced in the reaction vessel containing ethylenediamine, and then, the mixture was reacted at room temperature for 3 hours. After the reaction was completed, the solvent was removed using a rotary evaporator (Buchi, R-2055), and the residue was dissolved again in a small amount of chloroform, and then, extracted with a NaCl saturated solution and NaCO₃ to recover a chloroform layer.

And then, the solvent was removed with a rotary evaporator, and the residue was dissolved in chloroform, and then, silica-gel chromatography was conducted to separate. To a fraction eluted with chloroform:methanol=9:1(v/v), a hydrochloric acid solution was added in 50 equivalents of cholesteryl chloroformate, and methanol was gradually added until a single phase was formed so as to form AC-cholesterol hydrochloride.

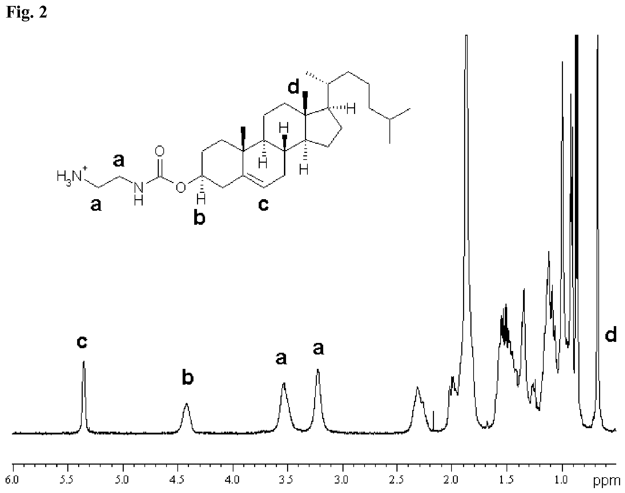

The solvent was completely removed by heating and distillation under reduced pressure with a rotary evaporator. The AC-cholesterol hydrochloride was dissolved in methanol of 60°C, and then, cooled to 4°C to obtain recrystal. The yield was about 53%. Synthesis and purity of AC-cholesterol were confirmed by ¹H-NMR, and the result is shown in Fig. 2. The purity was 99% or more.

### [Example 2] Synthesis of MC-cholesterol(3β[N-(N'-monomethylaminoethane)carbamoyl]cholesterol)

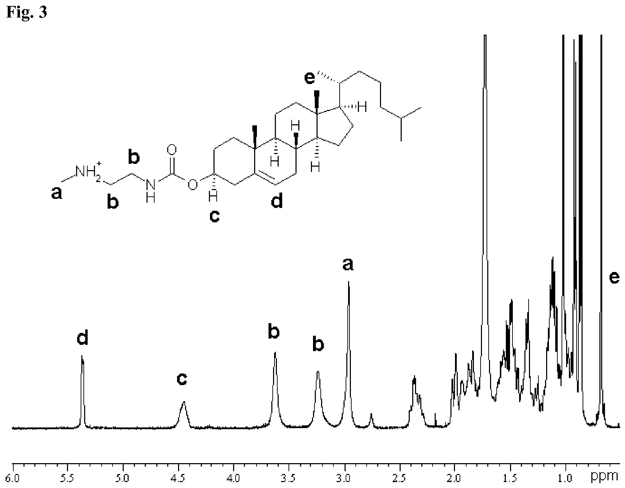

MC-cholesterol was synthesized and purified by the same method as Example 1, except that N-metheylethylenediamine (Sigma-Aldrich) was used in 10 equivalents of cholesteryl chloroformate instead of ethylenediamine. The yield was 62%. Synthesis and purity of AC-cholesterol were confirmed by ¹H-NMR, and the result is shown in Fig. 3. The purity was 99% or more.

### [Example 3] Polymerization of mPEG-PLA (monomethoxy ethylene glycol-polylactide) block copolymer (A-B) (molecular weight 2,000-1,750 Dalton)

5g of monomethoxy polyethylene glycol (molecular weight 2,000 Dalton or less, NOF corporation) was added to a 100ml two-necked round bottom flask, and heated to 100°C under reduced pressure (1mmHg) for 3 hours to dehydrate. Dry nitrogen was filled in the reaction flask, and a reaction catalyst of stannous octoate (Sn(Oct)₂, Sigma-Aldrich) was injected in the amount of 0.1wt% of lactide (5 mg). The reaction mixture was agitated for 30 minutes, and pressure was reduced to 1mmHg at 110 °C for 1 hour to remove toluene which is a solvent dissolving the catalyst. Purified lactide (5g, Purac) was added, and the mixture was heated to 130 °C for 12 hours. The formed polymer was dissolved in ethanol, and diethylether was added to precipitate a polymer. The precipitated polymer was dried in a vacuum oven for 48 hours.

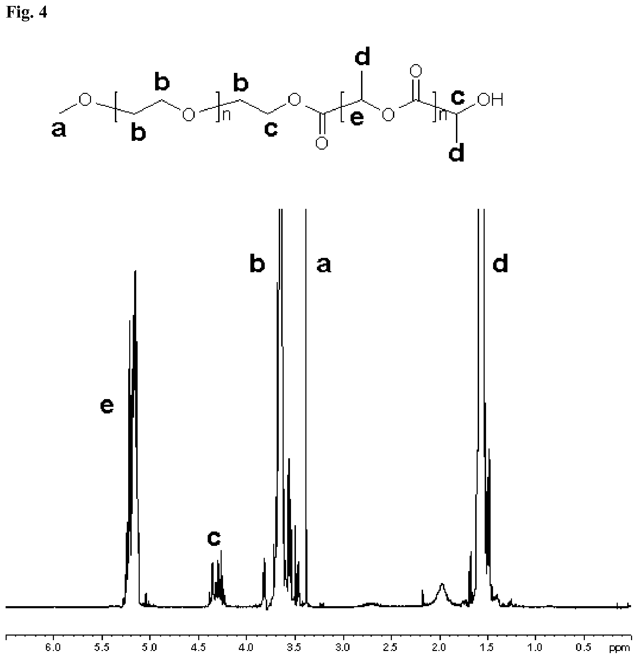

The obtained mPEG-PLA has number average molecular weight of 2,000-1,750 Dalton, and it was confirmed to be of A-B type by ¹H-NMR in Fig. 4.

### [Example 4] Polymerization of mPEG-PLA (monomethoxy polyethylene glycol-polylactide) block copolymer (A-B) (molecular weight 5,000-4,000 Dalton)

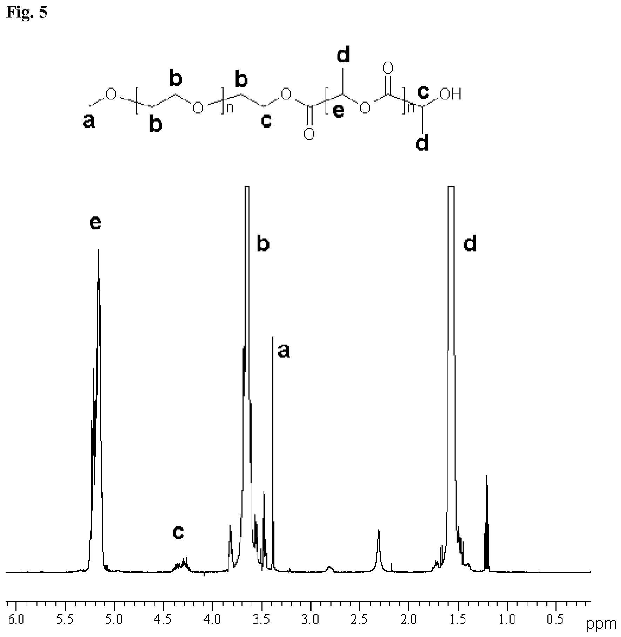

A mPEG-PLA block copolymer having number average molecular weight of 5,000-4,000 Dalton was synthesized by the same method as Example 3, using monomethoxy polyethylene glycol (molecular weight 5,000 Dalton or less, NOF corporation). The ¹H-NMR measurement results of the obtained mPEG-PLA block copolymer is shown in Fig. 5. As shown in Fig. 5, it is confirmed that the prepared mPEG-PLA block copolymer is of A-B type.

### [Example 5] Polymerization of mPEG-PLA-tocopherol (molecular weight 2,000-1,750-530 Dalton)

200ml of acetonitrile (CAN) was used as a reaction solvent, and 26.4 mmol of mPEG-PLA of Example 3 with number average molecular weight of 2,000-1,750 Dalton and 31.68mmol of tocopherol succinate (Sigma-Aldrich) as reactants, and 31.68mmol of dicyclohexyl carbodiimide (DCC, Sigma-Aldrich) and 3.168mmol of dimethylaminopyridine (DAMP, Sigma-Aldrich) as catalysts were introduced to synthesize at room temperature for 24 hours. The acetonitrile solution in which the reaction product was dissolved was filtered with a glass filter to remove dicyclohexylcarbourea (DCU) produced during the reaction.

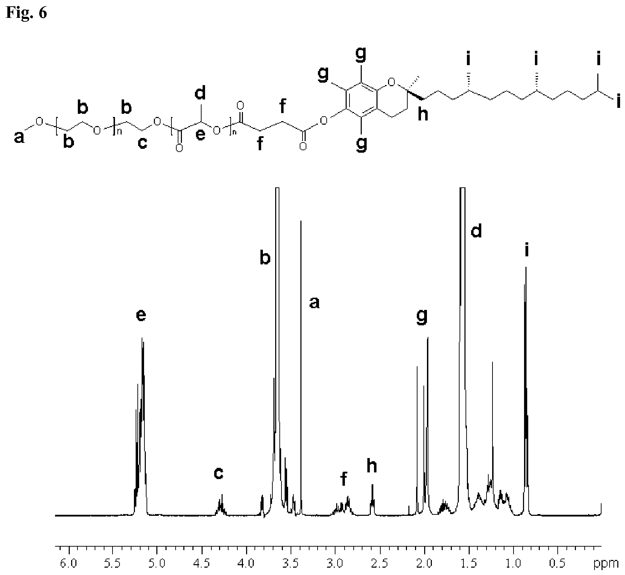

As a primary purification, the filtered acetonitrile solution was precipitated in a cool mixed solvent of diethylether:hexane=3:7(v/v) to recrystallize a polymer. The obtained polymer was dissolved again in an acetonitrile solution and precipitated in a mixed solvent of diethylether:hexane=3:7(v/v) to conduct a secondary purification. The purified polymer was vacuum dried to obtain white powder particles. In the ¹H-NMR analysis of Fig. 6, purity was 97% or more, and yield was 92.7%.

### [Example 6] Polymerization of mPEG-PLA-tocopherol (molecular weight 5,000-4,000-530 Dalton)

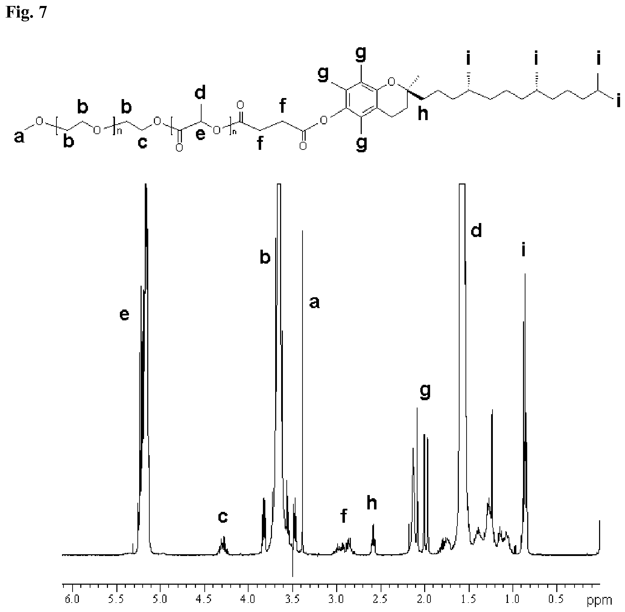

A mPEG-PLA-tocopherol was polymerized by the same method as Example 5, using mPEG-PLA of Example 4 with number average molecular weight of 5,000-4,000 Dalton. In the ¹H-NMR analysis of Fig. 7, purity was 97% or more, and the yield was 94.2%.

### [Example 7] Polymerization of Anisamide-PEG-PLA

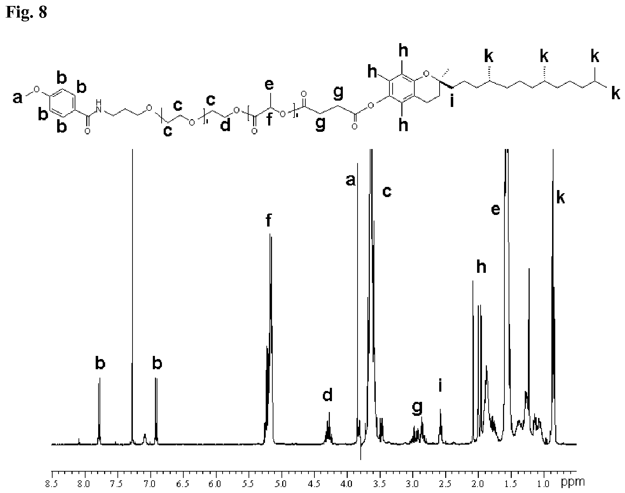

0.1 g (660µmol) of anisic acid (4-methoxybenzoic acid, Sigma-Aldrich), 0.146g (710µmol) of dicyclohexylcarbodimide (Sigma-Aldrich), and 0.081g (710µmol) of N-hydrosuccinimide (NHS, Sigma-Aldrich) were dissolved in a mixed solvent of acetonitrile:dimethylformamide (DMF)=2:1(v/v) and reacted for 24 hours to synthesize anisic acid-NHS ester (AA-NHS), and then, reaction by product of dicyclohexylcarbourea was filtered to remove. 0.519g(260µmol) of H₂N-PEG-OH (Mn=2,000, NOF corporation) was dissolved in 2ml of acetonitrile and 1.5 equivalents of AA-NHS was added, and then, the reaction mixture was reacted at room temperature for 24 hours to synthesize anisamide-PEG (AA-PEG). The process of precipitating the reactant in cool diethylether to recrystallize AA-PEG was repeated twice to purify AA-PEG. The process of polymerizing AA-PEG-PLA-tocopherol from AA-PEG was performed by the same method as Examples 5 and 6. In the ¹H-NMR analysis, introduction rate of anisamide was 90.2%, and the result is shown in Fig. 8.

### [Example 8] Preparation of siRNA/cationic lipid complex

A siRNA/cationic lipid complex was prepared using Bligh & Dyer extraction method (Bligh, EG., Dyer, WJ, A rapid method of total lipid extraction and purification, Can. J. Biochem. Physiol 37 (1959) 911-937). 5µg of the siRNA was used, and as the cationic lipid, AC-cholesterol, MC-cholesterol and TC-cholesterol (Sigma Aldrich) of Examples 1 and 2 were respectively used 0, 1, 2, 4, 8, and 16 times of the moles of siRNA phosphate groups (N/P ratio (the ratio of cation of the cationic lipid to the phosphate groups of siRNA)=0, 1, 2, 4, 8 and 16).

GFP siRNA sequence (Dharmacon):
Sense strand: 5'-GCAAGCUGACCCUGAAGUUdTdT-3' (Sequence ID No. 1)
Antisense strand: 5'-AACUUCAGGGUCAGCUUGCdTdT-3' (Sequence ID No. 2)

100µl of the siRNA aqueous solution, 100µl of the cationic lipid chloroform solution and 120µl of methanol were mixed in the above N/P ratio to form a monophase (Bligh & Dyer monophase), 100µl of distillated water and 100µl of chloroform were added to separate the phases. The amount of siRNA in the aqueous solution layer and the chloroform layer were quantified with a Ribogreen reagent (Invitrogen).

**[Table 1]**

| | Ratio of the amount of siRNA existing in each phase to the amount of siRNA introduced after phase shift (%) | | | | | |
|---|---|---|---|---|---|---|
| N/P ratio | AC-cholesterol | | MC-cholesterol | | TC-cholesterol | |
| | Aqueousphase | organicphase | Aqueousphase | Organicphase | aqueousphase | Organicphase |
| 0 | 100.8 | 0 | 95.4 | 0 | 99.1 | 0 |
| 1 | 37.7 | 70.9 | 93.3 | 0 | 0 | 97.7 |
| 2 | 0 | 100.1 | 27.5 | 72.7 | 0 | 98.9 |
| 4 | 0 | 106.1 | 0 | 102.2 | 0 | 97.9 |
| 8 | 0 | 105.6 | 0 | 102.8 | 0 | 96.8 |
| 16 | 0 | 114.7 | 0 | 105.4 | 0 | 98.2 |

Referring to Table 1, it is confirmed that the cationic lipids form a complex with siRNA and the siRNA/cationic lipid complex is phase-shifted to the organic solvent layer.

### [Example 9] Preparation of siRNA/AC-cholesterol/mPEG-PLA polymeric micelle

A siRNA/cationic lipid complex was prepared according to the method of Example 8. The ratio of the cation of AC-cholesterol to the phosphate group of siRNA (N/P ratio) was 6. After phase separation, a chloroform layer was separately collected and added to mPEG-PLA of Example 3 such that the ratio of siRNA/AC-cholesterol complex to mPEG-PLA (molecular weight 2,000-1,750 Dalton) may be 0.51wt%, and then, the mixture was moved into an 1-necked round flask, and distilled under reduced pressure in a rotary evaporator to remove the solvent. 300 µL of distillated water was added to the flask, and gently shaken to dissolve, thereby preparing a siRNA/AC-cholesterol/mPEG-PLA polymeric micelle delivery system.

### [Example 10] Preparation of siRNA/AC-cholesterol/mPEG-PLA-tocopherol polymeric micelle

A siRNA/AC-cholesterol/mPEG-PLA-tocopherol polymeric micelle delivery system was prepared by the same method of Example 9, except using mPEG-PLA-tocopherol (molecular weight 2,000-1,750-530 Dalton) of Example 5 instead of mPEG-PLA. The ratio of the siRNA/AC-cholesterol complex to mPEG-PLA-tocopherol was 0.51 wt%.

### [Example 11] Preparation of siRNA/AC-cholesterol/mPEG-PLA-tocopherol polymeric micelle

To an one-necked round flask, 46µg of AC-cholesterol (N/P ratio = 6) and ethanol were introduced and completely dissolved at room temperature, and then, 5µg of siRNA of Example 8 was added and mixed. 9 mg of mPEG-PLA-tocopherol (molecular weight 5,000-4,000-530 Dalton) of Example 6 was added thereto, and agitated at 60 °C for 5 minutes. The ratio of the siRNA/AC-cholesterol complex to mPEG-PLA-tocopherol was controlled to 0.57 wt%.

The mixture was distilled under reduced pressure in a rotary evaporator to remove the solvent. 300 µL of distillated water was added to the flask, and gently shaken to dissolve, thereby preparing a siRNA/AC-cholesterol/ mPEG-PLA polymeric micelle delivery system.

### [Example 12] Preparation of VEGF siRNA or siRNA-cholesterol/AC-cholesterol/mPEG-PLA-tocopherol polymeric micelle

VEGF siRNA of the following Sequence ID Nos. 3 and 4 and VEGF siRNA-cholesterol which has a sequence identical to the above sequence but includes cholesterol covalently bonded at 3'end were purchased from Samchully Pharm., and VEFG siRNA and VEGF siRNA-cholesterol polymeric micelle delivery system was prepared by the same method as Example 11.

VEGF siRNA (Dharmacon):
Sense strand: 5'-GGAGUACCCUGAUGAGAUCdTdT-3' (Sequence ID No. 3),
Antisense strand: 5'-GAUCUCAUCAGGGUACUCCdTdT-3' (Sequence ID No. 4)

### [Example 13] Preparation of siRNA/AC-cholesterol/mPEG-PLA-tocopherol/dioleylphosphatidyl-ethanolamine (DOPE) polymeric micelle

In the composition of Example 11, 34µg of DOPE (Avanti polar lipids) was additionally added together with the polymer to prepare a DOPE-containing siRNA polymeric micelle delivery system by the same method as Example 11.

### [Experimental Example 1] Measurement of the size of siRNA/cationic lipid/amphiphilic block copolymeric micelle and confirmation of siRNA enclosure

To confirm whether the siRNA/cationic lipid containing amphiphilic block copolymer forms a nanoparticle, the sizes of siRNA/AC-cholesterol/mPEG-PLA polymeric micelle and siRNA/AC-cholesterol/mPEG-PLA-tocopherol polymeric micelle were measured by DLS (Dynamic Light Scattering) method and described in Table 2.

A helium-neon laser with an output of 10 mV and wavelength of 638 nm was used as a light source, incident light of 90 °C was used, and the experiment was conducted at 25 °C. The measurement and analysis were conducted using an ELS-8000 equipment of Photal Otsuka Electronics Co. Ltd.

**[Table 2]**

| | Kind of polymer | Weight average particle size |
|---|---|---|
| Example 9 | siRNA/AC-cholesterol/mPEG-PLA | 27.6±16.9nm |
| Example 10 | siRNA/AC- cholesterol/mPEG-PLA-tocopherol | 26.8±7.2 nm |
| Example 11 | siRNA/AC- cholesterol/mPEG-PLA- tocopherol | 54.5±17.0nm |
| Example 12 | VEGF siRNA- cholesterol/AC-cholesterol/mPEG-PLA-tocopherol | 60.0±15.4 nm |
| Example 13 | siRNA/AC-cholesterol/mPEG-PLA-tocopherol /DOPE | 82.4±28.5 nm |

The siRNA was quantified in the prepared siRNA/cationic lipid containing amphiphilic block copolymeric micelle by a modified Bligh & Dyer extraction method.

The polymeric micelle delivery systems prepared in each Example was dissolved in 50 mM sodium phosphate, 75 mM NaCl (pH 7.5), and a Bligh & Dyer monophase was formed, and then, extracted with 100 mM sodium phosphate, 150 mM NaCl (pH 7.5) to quantify the siRNA of the aqueous solution layer with a Ribogreen reagent (Invitrogen).

As result of measurement, 90% or more of the siRNA amount could be extracted.

### [Experimental Example 2] Blood stability measurement of siRNA/AC-cholesterl/mPEG-PLA-tocopherol polymeric micelle

To examine how safely the siRNA/AC-cholesterol/mPEG-PLA-tocopherolpolymeric micelle protects siRNA in blood, half life of siRNA was measured in blood serum. The polymeric micelle of Example 10 (polymeric micelle 1) and the polymeric micelle of Example 11 (polymeric micelle 2) were cultured at 37°C, in 50% blood serum for the time described in Table 3, and then, the amount of siRNA was quantified to calculate the half life as follows.

To measure the total amount of siRNA of the polymeric micelle, modified Bligh & Dyer method as Experimental Example 1 was performed. The measurement results are described in the following Table 3.

**[Table 3]**

| Time (min) | Non-enclosed siRNA(%) | siRNA(%) of polymeric micelle 1 | siRNA(%) of polymeric micelle 2 |
|---|---|---|---|
| 30 | 32.4 | 63.6 | 92.5 |
| 60 | 29.1 | 57.6 | 74.7 |
| 120 | 19.8 | 46.9 | 58.1 |
| 240 | 8.8 | 31.2 | 47.4 |

Referring to Table 3, it is confirmed that the half life of non-enclosed siRNA is 28.4 minutes, while the half life of the siRNA enclosed in the polymeric micelle 1 of Example 10 is 126 minutes and the half life of the siRNA enclosed in the polymeric micelle 2 of Example 11 is 192.5 minutes, and that the half lives of siRNAs increased respectively 4.4 times and 6.8 times compared to the non-enclosed siRNA. It can be seen from the Table 3 that siRNA may be stabilized in blood by enclosing siRNA in a polymeric micelle.

### [Experimental Example 3] Stability evaluation of siRNA or siRNA-cholesterol/AC-cholesterol/mPEG-PLA-tocopherol polymeric micelle to RNase

It was examined how safely a siRNA or siRNA-cholesterol/AC-cholesterol/mPEG-PLA-tocopherol containing composition protects siRNA to RNase. The polymeric micelle of Example 11 (polymeric micelle 2) and the siRNA-cholesterol polymeric micelle of Example 12 (polymeric micelle 3) were cultured with 10U RNase VI (Promega) for the time described in Table 4, and then, the amount of siRNA was quantified by the same method as Experimental Example 1. The measurement result is described in the following Table 4.

**[Table 4]**

| Time (min) | Amount of non-enclosed siRNA(%) | siRNA amount of polymeric micelle 2 (siRNA) (%) | Amount of non-enclosed siRNA-cholesterol (%) | siRNA amount of polymeric micelle 3 (siRNA-cholesterol) (%) |
|---|---|---|---|---|
| 40 | 0 | 58.6 | 3.9 | 103.0 |
| 70 | 0 | 53.2 | 3.7 | 101.7 |
| 130 | 0 | 40.3 | 2.4 | 103.4 |

Referring to Table 4, it can be seen that the non-enclosed siRNA was completely degrade within 40 minutes after RNase treatment, while if the siRNA is enclosed in the polymeric micelle, about 40% remained stably even 130 minutes after RNase treatment. Meanwhile, it can be seen that siRNA-cholesterol has slightly higher stability than siRNA in non-enclosed states, and that if the siRNA-cholesterol is enclosed in the polymeric micelle (polymeric micelle 3), stability much increased compared to the siRNA enclosed in the polymeric micelle (polymeric micelle 2). Thus, it can be seen from the Table 4 that siRNA could be stabilized to RNase by enclosing siRNA in the polymeric micelle, and that the effect is more exhibited for siRNA-cholesterol.

### [Experimental Example 4] Evaluation of activity of siRNA/AC-cholesterol/mPEG-PLA-tocopherol polymeric micelle (protein level)

An A549 GFP cell line expressing GFP (Green fluorescence protein) [commonly prepared from A549 cell line (ATCC)] was treated with the GFP siRNA/AC-cholesterol/mPEG-PLA-tocopherol polymeric micelle of Example 10 and 11. And then, intracellular delivery capacity of the polymeric micelle was measured by measuring fluorescence shown by the expression of GFP protein.

The compositional ratio of the GFP siRNA/AC-cholesterol/mPEG-PLA-tocopherol containing composition is as described in the following Table 5.

**[Table 5]**

| Example | composition | N/P Ratio | mPEG-PLA-tocopherol molecular weight | siRNA/AC-cho lesterol amount (weight ratio) compared to mPEG-PLA-tocopherol |
|---|---|---|---|---|
| 10 | 1 | 6 | 2,000-1,750-530 | 0.648 |
| | 2 | 4 | 2,000-1,750-530 | 0.669 |
| | 3 | 3 | 2,000-1,750-530 | 0.700 |
| 11 | 4 | 6 | 5,000-4,000-530 | 0.648 |
| | 5 | 4 | 5,000-4,000-530 | 0.669 |
| | 6 | 3 | 5,000-4,000-530 | 0.700 |

1 x 10⁴ cells were divided on a 96-well cell plate, and after 24 hours, treated with 30 Nm of siRNA in the presence of 10% blood serum for 24 hours, and then, the medium was changed. After 24 hours, GFP fluorescence was measured with an ELISA reader (excitation wavelength: 485/20 nm, emission wavelength: 528/20 nm). The measurement result is shown in the following Table 6. Control was treated with phosphate buffered saline only.

**[Table 6]**

| Example | composition | GFP Fluorescence (%) | Cell viability (%) | GFP fluorescence /cell viability (%) |
|---|---|---|---|---|
| | Control | 98.4 | 99.8 | 98.6 |
| 10 | 1 | 48.1 | 89.9 | 53.6 |
| | 2 | 63.4 | 101.1 | 62.8 |
| | 3 | 68.2 | 101.1 | 67.6 |
| 11 | 4 | 67.7 | 90.9 | 74.5 |
| | 5 | 62.2 | 96.1 | 64.8 |
| | 6 | 80.7 | 95.4 | 84.6 |

Table 6 shows results obtained by measuring GFP fluorescence, and then, calculating cell viability by SRB assay, and dividing the GFP fluorescence value by the cell viability. It can be seen from Table 6 that GFP protein expression was inhibited about 30∼40%.

### [Experimental Example 5] Evaluation of activity (mRNA level) of siRNA/AC-cholesterol/mPEG-PLA-tocopherol polymeric micelle

For the compositions 1 to 3 of Experimental Example 4, the activity of siRNA/AC-cholesterol/mPEG-PLA-tocopherol polymeric micelle was confirmed at mRNA level. The polymeric micelle was treated under the same conditions as Experimental Example 4, except that the administration concentration of siRNA was varied to 15 nM and 30 nM. Cells were treated with the polymeric micelle, and after 48 hours, GFP mRNA and GAPDH mRNA were subjected to Quantitive RT-PCR to comparatively quantify GFP mRNA. Control was treated with phosphate buffered saline only. The result of quantification is shown in the following Table 7

**[Table 7]**

| Composition | Administration concentration (nM) | GFP mRNA expression (%) |
|---|---|---|
| Control | 0 | 100.0% |
| 1 | 15 | 40.1% |
| | 30 | 4.6% |
| 2 | 15 | 66.9% |
| | 30 | 6.1% |
| 3 | 15 | 71.2% |
| | 30 | 10.3% |

Table 7 shows the activities of tocopherol polymeric micelle delivery systems examined by the expression amount of mRNA. It can be seen from the Table 7 that the amount of GFP mRNA decreased in proportion to the administration amount, and that GFP mRNA was inhibited 90% or more at 30 nM.

### [Experimental Example 6] Activity comparison experiment of siRNA polymeric micelle and lipofectamine

The activity of siRNA/AC-cholesterol/mPEG-PLA-tocopherol polymeric micelle and the activity of lipofectamine (Invitrogen) commercially used for cell delivery of nucleic acid were compared at protein level. The experiment was conducted by the same method as Experimental Example 4 for the composition 1 of Experimental Example 4. Control was treated with phosphate buffered saline only. The results are shown in the following Table 8.

**[Table 8]**

| composition | GFP fluorescence(%) | Cell viability (%) | GFP fluorescence/cell viability (%) |
|---|---|---|---|
| Control | 98.4 | 99.8 | 98.6 |
| Composition 1 of Experimental Example 4 | 57.0 | 99.5 | 57.3 |
| Lipofectamine | 47.9 | 73.0 | 65.5 |

Table 7 shows the results of comparison of activities of siRNA polymeric micelle and lipofectamine examined by the amount of protein expression. It can be seen from the Table 8 that siRNA polymer inhibited expression of GFP protein with the similar level to lipofectamine while exhibiting higher cell viability. This means that siRNA polymeric micelle delivery system has more excellent activity compared to toxicity than lipofectamine.

### [Experimental Example 7] In vivo activity of siRNA/AC-cholesterol/mPEG-PLA-tocopherol polymeric micelle

It was confirmed whether siRNA/AC-cholesterol/mPEG-PLA-tocopherolpolymeric micelle can inhibit target gene VEGF (vascular endothelial growth factor) of used siRNA in the living body.

A nude mouse (provided by Central Lab. Animal Inc.) was subcutaneously injected with A549 lung cancer cell line (ATCC) to prepare a cancer-induced mouse. The cancer model mouse was intravenously injected with the VEGF siRNA/AC-cholesterol/mPEG-PLA-tocopherol polymeric micelle of Example 12 at a dose of 1.5 mg/kg, and after 48 hours, cancer tissue was extracted. The extracted cancer tissue was pulverized and the amount of VEGF protein was analyzed by ELISA. The ELISA was conducted according to the instruction of kit manufacturer (R&D systems). As control, saline solution was injected. The results are shown in Table 9.

**[Table 9]**

| Group | Individual | VEGF concentration (pg/ml) | Relative amount (%) | average (%) |
|---|---|---|---|---|
| Control | #1 | 820.6 | 127.5 | 100.0 |
| | #2 | 475.0 | 73.8 | |
| | #3 | 610.5 | 94.9 | |
| | #4 | 668.3 | 103.8 | |
| VEGF siRNA polymeric micelle | #1 | 342.7 | 53.3 | 57.2 |
| | #2 | 344.9 | 53.6 | |
| | #3 | 356.8 | 55.4 | |
| | #4 | 427.5 | 66.4 | |

Table 9 shows inhibition rate of target gene in cancer tissue after intravenous injection of siRNA polymeric micelle delivery system in a caner model mouse. The siRNA polymeric micelle delivery system inhibited the amount of VEGF protein about 43% in the cancer tissue. It can be seen from the Table 9 that systemic delivery of siRNA may be enabled with the siRNA polymeric micelle delivery system.

### [Experimental Example 8] In vivo activity of siRNA-cholesterol/AC-cholesterol/mPEG-PLA-tocopherol polymeric micelle

The experiment was conducted by the same method as Experimental Example 7, except using VEGF siRNA-cholesterol/AC-cholesterol/mPEG-PLA-tocopherol polymeric micelle of Example 12, and then, the concentration of VEGF was analyzed. As control, a saline solution was used. The results are shown in Table 10.

**[Table 10]**

| Group | individual | VEGF concentration (pg/ml) | Relative amount (%) | average (%) |
|---|---|---|---|---|
| Control | #1 | 438.6 | 82.9 | 100.0 |
| | #2 | 403.7 | 76.3 | |
| | #3 | 745.6 | 140.9 | |
| siRNA -cholesterol polymeric micelle of Example 12 | #1 | 218.9 | 41.4 | 32.0 |
| | #2 | 173.1 | 32.7 | |
| | #3 | 115.3 | 21.8 | |

Table 10 shows inhibition rate of target gene in the cancer tissue after intravenous injection of siRNA-cholesterol polymeric micelle delivery system in a cancer model mouse. The siRNA-cholesterol polymeric micelle delivery system inhibited the amount of VEGF protein about 68% in the cancer tissue. It can be seen from the Table 10 that systemic delivery of siRNA may be enabled with the siRNA-cholesterol polymeric micelle delivery system.

### [Experimental Example 9] Evaluation of activity (protein level) of siRNA/AC-cholesterol/mPEG-PLA-tocopherol/DOPE polymeric micelle

The effect of addition of DOPE to siRNA/AC-cholesterol/mPEG-PLA-tocopherol polymeric micelle on the activity was examined. A polymeric micelle comprising DOPE was prepared by the same method as Example 13 with the VEGF siRNA sequence of Example 12. A549 cell lines were respectively treated with the above micelle and the VEGF siRNA/AC-cholesterol/mPEG-PLA-tocopherol polymeric micelle of Example 12 by the same method as Experimental Example 4. The medium was recovered, and the concentration of released VEGF in the medium was measured by the method described in Experimental Example 7, and corrected with respect to control treated with phosphate buffered saline only. The measurement results are shown in the following Table 11.

**[Table 11]**

| | control | siRNA polymeric micelle | DOPE containing siRNA polymeric micelle |
|---|---|---|---|
| VEGF concentration | 100% | 79.1% | 38.8% |
| siRNA activity (VEGF inhibition rate) | 0% | 20.9% | 61.2% |

Table 11 shows quantification of the concentration of VEGF protein released in the medium after treating the siRNA polymeric micelle. It can be seen from the Table 11 that siRNA activity largely increases from 20.9% to 61.2% by adding DOPE to the siRNA polymeric micelle.

## Claims

1. A composition for delivery of an anionic drug consisting of
an anionic drug as an active ingredient;
a cationic lipid; and
an amphiphilic block copolymer,
optionally at least one fusogenic lipid, and
an aqueous solution or an assistant agent for freeze drying,
wherein the anionic drug forms a complex with the cationic lipid, and the complex is entrapped in the core-shell type polymeric micelle structure of the amphiphilic block copolymer of an A-B type di-block copolymer comprising of a hydrophilic A block forming a shell and having a number average molecular weight of 200 to 50,000 Dalton and a hydrophobic B block forming a core and having a number average molecular weight of 50 to 50,000 Dalton in an aqueous solution, and
wherein the content of the amphiphilic block copolymer is 40-99.98 wt% based on the total dry weight of the composition; and
tocopherol, cholesterol, or C10-C24 fatty acid is optionally chemically conjugated to a hydroxyl group of the hydrophobic block end ,
wherein the hydrophilic A block is polyalkyleneglycol, and the hydrophobic B block is one or more selected from the group consisting of polyester, polyanhydride, polyamino acid, polyorthoester, and polyphosphazine.

2. The composition of claim 1, wherein the anionic drug is nucleic acid material.

3. The composition of claim 2, wherein the nucleic acid material is one or more selected from the group consisting of RNA, DNA, siRNA (short interfering RNA), aptamer, antisense ODN (oligodeoxynucleotide), antisense RNA, ribozyme, and DNAzyme.

4. The composition of claim 2, wherein the nucleic acid material is modified by modifying at least one end of the nucleic acid material with one or more selected from the group consisting of cholesterol, tocopherol, and C10-C24 fatty acid.

5. The composition of claim 1, wherein the cationic lipid is one or more selected from the group consisting of N,N-dioleyl-N,N-dimethylammoniumchloride (DODAC), N,N-distearyl-N,N-dimethylammoniumbromide (DDAB), N-(1-(2,3-dioleoyloxy)propyl-N,N,N-trimethylammoniumchloride (DOTAP), N,N-dimethyl-(2,3-dioleoyloxy)propylamine (DODMA), 1,2-diacyl-3-trimethylammonium-propane (TAP), 1,2-diacyl-3-dimethylammonium-propane (DAP), 3β-[N-(N',N',N'-trimethylaminoethane)carbamoyl]cholesterol (TC-cholesterol), 3 β[N-(N',N'-dimethylaminoethane)carbamoyl]cholesterol (DC-cholesterol), 3β[N-(N'-monomethylaminoethane)carbamoyl] cholesterol (MC-cholesterol), 3β[N-(aminoethane)carbamoyl]cholesterol (AC-cholesterol), cholesteryloxypropane-1-amine (COPA), N-(N'-aminoethane)carbamoylpropanoic tocopherol (AC-tocopherol), and N-(N'-methylaminoethane)carbamoylpropanoic tocopherol (MC-tocopherol).

6. The composition of claim 1, wherein the ratio of quantities of electric charges of the cationic lipid (N) to the anionic drug (P) (N/P) is 0.1 to 128.

7. The composition of claim 1, wherein the ratio of the weight of the complex of the anionic drug and the cationic lipid (a) to the weight of the amphiphilic block copolymer (b) (a/b X 100) is 0.001 to 100 wt%.

8. The composition of claim 1, wherein the fusogenic lipid is at least one selected from the group consisting of phospholipid, cholesterol, and tocopherol.

9. The composition of claim 8, wherein the phospholipid is one or more selected from the group consisting of phosphatidylethanolamine (PE), phosphatidylcholine (PC), and phosphatidic acid.

10. The composition of claim 8, wherein the fusogenic lipid is one or more selected from the group consisting of dilauroyl phosphatidylethanolamine, dimyristoyl phosphatidylethanolamine, dipalmitoyl phosphatidylethanolamine, distearoyl phosphatidylethanolamine, dioleoyl phosphatidylethanolamine, dilinoleoyl phosphatidylethanolamine, 1-palmitoyl-2-oleoyl phosphatidylethanolamine, 1,2-diphytanoyl-3-sn-phosphatidylethanolamine, dilauroyl phosphatidylcholine, dimyristoyl phosphatidylcholine, dipalmitoyl phosphatidylcholine, distearoyl phosphatidylcholine, dioleoyl phosphatidylcholine, dilinoleoyl phosphatidylcholine, 1-palmitoyl-2-oleoyl phosphatidylcholine, 1,2-diphytanoyl-3-sn-phosphatidylcholine, dilauroyl phosphatidic acid, dimyristoyl phosphatidic acid, dipalmitoyl phosphatidic acid, distearoyl phosphatidic acid, dioleoyl phosphatidic acid, dilinoleoyl phosphatidic acid, 1-palmitoyl-2-oleoyl phosphatidic acid, 1,2-diphytanoyl-3-sn-phosphatidic acid, cholesterol, and tocopherol.

11. A method of preparing a composition for delivery of an anionic drug consisting of an anionic drug, a cationic lipid, an amphiphilic block copolymer, optionally at least one fusogenic lipid, and an aqueous solution or an assistant agent for freeze drying, which method comprises:
(a) dissolving the anionic drug and the cationic lipid in a water-miscible organic solvent or a mixed solvent of an aqueous solution and an organic solvent, to separate the phases;
(b) separating the organic solvent layer of (a);
(c) mixing the organic solvent layer of (b) with the amphiphilic block copolymer and removing the organic solvent; and
(d) adding an aqueous solution to the mixture from which the organic solvent is removed, to form a micelle,
(e) optionally adding an assistant agent for freeze drying to freeze dry, to perform freeze drying,
wherein the anionic drug forms a complex with the cationic lipid, and the complex is entrapped in the core-shell type polymeric micelle structure of the amphiphilic block copolymer of an A-B type di-block copolymer comprising of a hydrophilic A block forming a shell and having a number average molecular weight of 200 to 50,000 Dalton and a hydrophobic B block forming a core and having a number average molecular weight of 50 to 50,000 Dalton in an aqueous solution, and wherein the content of the amphiphilic block copolymer is 40-99.98 wt% based on the total dry weight of the composition, and tocopherol, cholesterol, or C10-C24 fatty acid is optionally chemically conjugated to a hydroxyl group of the hydrophobic block end,
wherein the hydrophilic A block is polyalkyleneglycol, and the hydrophobic B block is one or more selected from the group consisting of polyester, polyanhydride, polyamino acid, polyorthoester, and polyphosphazine.

12. A method of preparing a composition for delivery of an anionic drug consisting of an anionic drug, a cationic lipid, an amphiphilic block copolymer, and optionally at least one fusogenic lipid, and an aqueous solution or an assistant agent for freeze drying, which method comprises:
(a') dissolving the anionic drug, the cationic lipid and the amphiphilic block copolymer in a water-miscible organic solvent or a mixed solvent of an aqueous solution and an organic solvent;
(b') removing the organic solvent layer of (a'); and
(c') adding an aqueous solution to the mixture of (b') from which the organic solvent is removed so as to form a micelle,
(d') optionally adding an assistant agent for freeze drying to freeze dry, to perform preeze drying,
wherein the anionic drug forms a complex with the cationic lipid, and the complex is entrapped in the core-shell type polymeric micelle structure of the amphiphilic block copolymer of an A-B type di-block copolymer comprising of a hydrophilic A block forming a shell and having a number average molecular weight of 200 to 50,000 Dalton and a hydrophobic B block forming a core and having a number average molecular weight of 50 to 50,000 Dalton in an aqueous solution, and wherein the content of the amphiphilic block copolymer is 40-99.98 wt% based on the total dry weight of the composition, and tocopherol, cholesterol, or C10-C24 fatty acid is optionally chemically conjugated to a hydroxyl group of the hydrophobic block end,
wherein the hydrophilic A block is polyalkyleneglycol, and the hydrophobic B block is one or more selected from the group consisting of polyester, polyanhydride, polyamino acid, polyorthoester, and polyphosphazine.

13. The method of claim 11 or claim 12,
wherein the fusogenic lipid is added in Step (c) or (a').

14. The method of claim 11 or claim 12, wherein the anionic drug is nucleic acid material.

15. The method of claim 11 or claim 12, wherein the cationic lipid is one or more selected from the group consisting of N,N-dioleyl-N,N-dimethylammoniumchloride (DODAC), N,N-distearyl-N,N-dimethylammoniumbromide (DDAB), N-(1-(2,3-dioleoyloxy)propyl-N,N,N-trimethylammoniumchloride (DOTAP), N,N-dimethyl-(2,3-dioleoyloxy)propylamine (DODMA), 1,2-diacyl-3-trimethylammonium-propane (TAP), 1,2-diacyl-3-dimethylammonium-propane (DAP), 3β-[N-(N',N',N'-trimethylaminoethane)carbamoyl]cholesterol (TC-cholesterol), 3 β[N-(N',N'-dimethylaminoethane)carbamoyl]cholesterol (DC-cholesterol), 3β[N-(N'-monomethylaminoethane)carbamoyl] cholesterol (MC-cholesterol), 3β[N-(aminoethane)carbamoyl]cholesterol (AC-cholesterol), cholesteryloxypropane-1-amine (COPA), N-(N'-aminoethane)carbamoylpropanoic tocopherol (AC-tocopherol), and N-(N'-methylaminoethane)carbamoylpropanoic tocopherol (MC-tocopherol).

16. The method of claim 11 or claim 12, wherein the ratio of the quantity of electric charges of the cationic lipid (N) to the anionic drug (P) (N/P) is 0.1 to 128.

17. The method of claim 11 or claim 12, wherein the ratio of the weight of the complex of the anionic drug and the cationic lipid (a) to the weight of the amphiphlic block copolymer (b) (a/b X 100) is 0.001 to 100 wt%.

## Patentansprüche

1. Zusammensetzung für die Bereitstellung eines anionischen Arzneimittels, bestehend aus
einem anionischen Arzneimittel als einen Wirkstoff;
ein kationisches Lipid; und
ein amphiphiles Blockcopolymer,
gegebenenfalls zumindest ein fusogenes Lipid, und
eine wässrige Lösung oder ein Unterstützungsmittel zum Gefriertrocknen,
wobei das anionische Arzneimittel einen Komplex mit dem kationischen Lipid bildet und der Komplex in der polymeren Kern-Schale-Mizellenstruktur des amphiphilen Blockcopolymers eines Di-Blockcopolymers vom A-B-Typ, umfassend einen hydrophilen A-Block, der eine Schale bildet und eine zahlenmittleres Molekulargewicht von 200 bis 50.000 Dalton aufweist, und einen hydrophoben B-Block, der einen Kern bildet und ein zahlenmittleres Molekulargewicht von 50 bis 50.000 Dalton aufweist, in einer wässrigen Lösung eingeschlossen ist, und wobei der Inhalt des amphiphilen Blockcopolymers 40-99,98 Gew.-% basierend auf dem Gesamttrockengewicht der Zusammensetzung beträgt; und
Tocopherol, Cholesterin oder C10-C24-Fettsäure gegebenenfalls mit einer Hydroxylgruppe des hydrophoben Blockendes chemisch konjugiert,
wobei der hydrophile A-Block Polyalkylenglycol ist und der hydrophobe B-Block eines oder mehrere ausgewählt aus der Gruppe bestehend aus Polyester, Polyanhydrid, Polyaminsäure, Polyorthoester und Polyphosphazin ist.

2. Zusammensetzung nach Anspruch 1, wobei das anionische Arzneimittel Nukleinsäurematerial ist.

3. Zusammensetzung nach Anspruch 2, wobei das Nukleinsäurematerial eines oder mehrere ist, ausgewählt aus der Gruppe bestehend aus RNA, DNA, siRNA (short interfering RNA), Aptamer, Antisense-ODN (Oligodeoxynucleotid), Antisense-RNA, Ribozym und DNAzym.

4. Zusammensetzung nach Anspruch 2, wobei das Nukleinsäurematerial durch Modifizieren von zumindest einem Ende des Nukleinsäurematerial mit einem oder mehreren, ausgewählt aus der Gruppe bestehend aus Cholesterin, Tocopherol und C10-C24-Fettsäure, modifiziert ist.

5. Zusammensetzung nach Anspruch 1, wobei das kationische Lipid eines oder mehrere ist, ausgewählt aus der Gruppe bestehend aus N,N-Dioleyl-N,N-dimethylammoniumchlorid (DODAC), N,N-Distearyl-N,N-dimethylammoniumbromid (DDAB), N-(1-(2,3-Dioleoyloxy)propyl-N,N,N-trimethylammoniumchlorid (DOTAP), N,N-Dimethyl-(2,3-dioleoyloxy)propylamin (DODMA), 1,2-Diacyl-3-trimethylammoniumpropan (TAP), 1,2-Diacyl-3-dimethylammoniumpropan (DAP), 3β-[N-(N',N',N'-Trimethylaminoethan)carbamoyl]cholesterin (TC-cholesterin), 3ß-[N-(N',N'-Dimethylaminoethan)carbamoyl]cholesterin (DC-Cholesterin), 3β-[N-(N'-Monomethylaminoethan)carbamoyl]cholesterin (MC-Cholesterin), 3β-[N-(Aminoethan)carbamoyl]cholesterin (AC-Cholesterin), Cholesteryloxypropan-1-amin (COPA), N-(N'-Aminoethan)carbamoylpropantocopherol (AC-Tocopherol) und N-(N'-Methylaminoethan)carbamoylpropantocopherol (MC-Tocopherol).

6. Zusammensetzung nach Anspruch 1, wobei das Verhältnis von Mengen von elektrischen Ladungen des kationischen Lipids (N) zu dem anionischen Arzneimittel (P) (N/P) 0,1 bis 128 beträgt.

7. Zusammensetzung nach Anspruch 1, wobei das Verhältnis des Gewichts des Komplexes des anionischen Arzneimittels und des kationischen Lipids (a) zu dem Gewicht des amphiphilen Blockcopolymers (b) (a/b X 100) 0,001 bis 100 Gew.-% beträgt.

8. Zusammensetzung nach Anspruch 1, wobei das fusogene Lipid zumindest eines ist, ausgewählt aus der Gruppe bestehend aus Phospholipid, Cholesterin und Tocopherol.

9. Zusammensetzung nach Anspruch 8, wobei das Phospholipid eines oder mehrere ist, ausgewählt aus der Gruppe bestehend aus Phosphatidylethanolamin (PE), Phosphatidylcholin (PC) und Phosphatidsäure.

10. Zusammensetzung nach Anspruch 8, wobei das fusogene Lipid eines oder mehrere ist, ausgewählt aus der Gruppe bestehend aus Dilauroylphosphatidylethanolamin, Dimyristoylphosphatidylethanolamin, Dipalmitoylphosphatidylethanolamin, Distearoylphosphatidylethanolamin, Dioleoylphosphatidylethanolamin, Dilinoleoylphosphatidylethanolamin, 1-Palmitoyl-2-oleoylphosphatidylethanolamin, 1,2-Diphytanoyl-3-sn-phosphatidylethanolamin, Dilauroylphosphatidylcholin, Dimyristoylphosphatidylcholin, Dipalmitoylphosphatidylcholin, Distearoylphosphatidylcholin, Dioleoylphosphatidylcholin, Dilinoleoylphosphatidylcholin, 1-Palmitoyl-2-oleoylphosphatidylcholin, 1,2-Diphytanoyl-3-sn-phosphatidylcholin, Dilauroylphosphatidsäure, Dimyristoylphosphatidsäure, Dipalmitoylphosphatidsäure, Distearoylphosphatidsäure, Dioleoylphosphatidsäure, Dilinoleoylphosphatidsäure, 1-Palmitoyl-2-oleoylphosphatidsäure, 1,2-Diphytanoyl-3-sn-phosphatidsäure, Cholesterin und Tocopherol.

11. Verfahren zum Herstellen einer Zusammensetzung zur Bereitstellung eines anionischen Arzneimittels, bestehend aus einem anionischen Arzneimittel, einem kationischen Lipid, einem amphiphilen Blockcopolymer, gegebenenfalls zumindest einem fusogenen Lipid und einer wässrigen Lösung oder einem Unterstützungsmittel zum Gefriertrocknen, wobei das Verfahren Folgendes umfasst:
(a) Auflösen des anionischen Arzneimittels und des kationischen Lipids in einem wassermischbaren organischen Lösungsmittel oder einem gemischten Lösungsmittel einer wässrigen Lösung und eines organischen Lösungsmittels, um die Phasen zu trennen;
(b) Trennen der organischen Lösungsmittelschicht aus (a);
(c) Mischen der organischen Lösungsmittelschicht aus (b) mit dem amphiphilen Blockcopolymer und entfernen des organischen Lösungsmittels; und
(d) Hinzufügen einer wässrigen Lösung zu dem Gemisch, aus dem das organische Lösungsmittel entfernt wird, um eine Mizelle zu bilden,
(e) gegebenenfalls Hinzufügen eines Unterstützungsmittels zum Gefriertrocken, um gefrierzutrockenen, um Gefriertrocknen durchzuführen,
wobei das anionische Arzneimittel einen Komplex mit dem kationischen Lipid bildet und der Komplex in der polymeren Kern-Schale-Mizellenstruktur des amphiphilen Blockcopolymers eines Di-Blockcopolymers vom A-B-Typ, umfassend einen hydrophilen A-Block, der eine Schale bildet und eine zahlenmittleres Molekulargewicht von 200 bis 50.000 Dalton aufweist, und einen hydrophoben B-Block, der einen Kern bildet und ein zahlenmittleres Molekulargewicht von 50 bis 50.000 Dalton aufweist, in einer wässrigen Lösung eingeschlossen ist, und wobei der Inhalt des amphiphilen Blockcopolymers 40-99,98 Gew.-% basierend auf dem Gesamttrockengewicht der Zusammensetzung beträgt, und Tocopherol, Cholesterin oder C10-C24-Fettsäure gegebenenfalls mit einer Hydroxylgruppe des hydrophoben Blockendes chemisch konjugiert,
wobei der hydrophile A-Block Polyalkylenglycol ist und der hydrophobe B-Block eines oder mehrere ausgewählt aus der Gruppe bestehend aus Polyester, Polyanhydrid, Polyaminsäure, Polyorthoester und Polyphosphazin ist.

12. Verfahren zum Herstellen einer Zusammensetzung zur Bereitstellung eines anionischen Arzneimittels, bestehend aus einem anionischen Arzneimittel, einem kationischen Lipid, einem amphiphilen Blockcopolymer, und gegebenenfalls zumindest einem fusogenen Lipid und einer wässrigen Lösung oder einem Unterstützungsmittel zum Gefriertrocknen, wobei das Verfahren Folgendes umfasst:
(a') Auflösen des anionischen Arzneimittels, des kationischen Lipids und des amphiphilen Blockcopolymers in einem wassermischbaren organischen Lösungsmittel oder einem gemischten Lösungsmittel einer wässrigen Lösung und eines organischen Lösungsmittels;
(b') Trennen Entfernen der organischen Lösungsmittelschicht aus
(a'); und
(c') Hinzufügen einer wässrigen Lösung zu dem Gemisch aus (b'), aus dem das organische Lösungsmittel entfernt wird, um so eine Mizelle zu bilden,
(d') gegebenenfalls Hinzufügen eines Unterstützungsmittels zum Gefriertrocken, um gefrierzutrockenen, um Gefriertrocknen durchzuführen, wobei das anionische Arzneimittel einen Komplex mit dem kationischen Lipid bildet und der Komplex in der polymeren Kern-Schale-Mizellenstruktur des amphiphilen Blockcopolymers eines Di-Blockcopolymers vom A-B-Typ, umfassend einen hydrophilen A-Block, der eine Schale bildet und eine zahlenmittleres Molekulargewicht von 200 bis 50.000 Dalton aufweist, und einen hydrophoben B-Block, der einen Kern bildet und ein zahlenmittleres Molekulargewicht von 50 bis 50.000 Dalton aufweist, in einer wässrigen Lösung eingeschlossen ist, und wobei der Inhalt des amphiphilen Blockcopolymers 40-99,98 Gew.-% basierend auf dem Gesamttrockengewicht der Zusammensetzung beträgt, und Tocopherol, Cholesterin oder C10-C24-Fettsäure gegebenenfalls mit einer Hydroxylgruppe des hydrophoben Blockendes chemisch konjugiert,
wobei der hydrophile A-Block Polyalkylenglycol ist und der hydrophobe B-Block eines oder mehrere ausgewählt aus der Gruppe bestehend aus Polyester, Polyanhydrid, Polyaminsäure, Polyorthoester und Polyphosphazin ist.

13. Verfahren nach Anspruch 11 oder Anspruch 12,
wobei das fusogene Lipid in Schritt (c) oder (a') hinzugefügt wird.

14. Verfahren nach Anspruch 11 oder Anspruch 12, wobei das anionische Arzneimittel Nukleinsäurematerial ist.

15. Verfahren nach Anspruch 11 oder Anspruch 12, wobei das kationische Lipid eines oder mehrere ist, ausgewählt aus der Gruppe bestehend aus N,N-Dioleyl-N,N-dimethylammoniumchlorid (DODAC), N,N-Distearyl-N,N-dimethylammoniumbromid (DDAB), N-(1-(2,3-Dioleoyloxy)propyl-N,N,N-trimethylammoniumchlorid (DOTAP), N,N-Dimethyl-(2,3-dioleoyloxy)propylamin (DODMA), 1,2-Diacyl-3-trimethylammoniumpropan (TAP), 1,2-Diacyl-3-dimethylammoniumpropan (DAP), 3β-[N-(N',N',N'-Trimethylaminoethan)carbamoyl]cholesterin (TC-cholesterin), 3ß-[N-(N',N'-Dimethylaminoethan)carbamoyl]cholesterin (DC-Cholesterin), 3β-[N-(N'-Monomethylaminoethan)carbamoyl]cholesterin (MC-Cholesterin), 3ß-[N-(Aminoethan)carbamoyl]Cholesterin (AC-Cholesterin), Cholesteryloxypropan-1-amin (COPA), N-(N'-Aminoethan)carbamoylpropantocopherol (AC-Tocopherol) und N-(N'-Methylaminoethan)carbamoylpropantocopherol (MC-Tocopherol).

16. Verfahren nach Anspruch 11 oder Anspruch 12, wobei das Verhältnis der Menge von elektrischen Ladungen des kationischen Lipids (N) zu dem anionischen Arzneimittel (P) (N/P) 0,1 bis 128 beträgt.

17. Verfahren nach Anspruch 11 oder Anspruch 12, wobei das Verhältnis des Gewichts des Komplexes des anionischen Arzneimittels und des kationischen Lipids (a) zu dem Gewicht des amphiphilen Blockcopolymers (b) (a/b X 100) 0,001 bis 100 Gew.-% beträgt.

## Revendications

1. Composition pour délivrer un médicament anionique consistant en
un médicament anionique en tant que principe actif ;
un lipide cationique ; et
un copolymère séquencé amphiphile,
facultativement au moins un lipide fusiogène, et
une solution aqueuse ou un agent assistant pour une lyophilisation,
dans lequel le médicament anionique forme un complexe avec le lipide cationique, et le complexe est piégé dans la structure de micelle polymère de type noyau-coque du copolymère séquencé amphiphile d'un copolymère di-séquencé de type A-B comprenant une séquence A hydrophile formant une coque et ayant un poids moléculaire moyen en nombre allant de 200 à 50 000 Dalton et une séquence B hydrophobe formant un noyau et ayant un poids moléculaire moyen en nombre de 50 à 50 000 Dalton dans une solution aqueuse, et
dans laquelle la teneur du copolymère séquencé amphiphile est de 40 à 99, 98 % en poids sur la base du poids sec total de la composition ; et
un tocophérol, un cholestérol, ou un acide gras en C10 à C24 est facultativement chimiquement conjugué à un groupe hydroxyle de l'extrémité de la séquence hydrophobe,
dans laquelle la séquence A hydrophile est un polyalkylèneglycol, et la séquence B hydrophobe est un ou plusieurs sélectionnés dans le groupe constitué d'un polyester, d'un polyanhydride, d'un polyacide aminé, d'un polyorthoester, et d'une polyphosphazine.

2. Composition selon la revendication 1, dans laquelle le médicament anionique est un matériau d'acide nucléique.

3. Composition selon la revendication 2, dans laquelle le matériau d'acide nucléique est un ou plusieurs sélectionnés dans le groupe constitué d'un ARN, d'un ADN, d'un ARNsi (ARN interférent court), d'un aptamère, d'un ODN antisens (oligodésoxynucléotide), d'un ARN antisens, d'un ribozyme et d'une ADNzyme.

4. Composition selon la revendication 2, dans laquelle le matériau d'acide nucléique est modifié en modifiant au moins une extrémité du matériau d'acide nucléique avec un ou plusieurs sélectionnés dans le groupe constitué du cholestérol, du tocophérol, et d'un acide gras en C10 à C24.

5. Composition selon la revendication 1, dans laquelle le lipide cationique est un ou plusieurs sélectionnés dans le groupe constitué du chlorure de N,N-dioléyl-N,N-diméthyl-ammonium (DODAC), du bromure de N,N-distéaryl-N,N-diméthylammonium (DDAB), du chlorure de N-(1-(2,3-dioléoyloxy)propyl-N,N,N-triméthylammonium (DOTAP), de la N,N-diméthyl-(2,3-dioléoyloxy)propylamine (DODMA), du 1,2-diacyl-3-triméthylammonium-propane (TAP), du 1,2-diacyl-3-diméthylammonium-propane (DAP), du 3β-[N-(N',N',N'-triméthylaminoéthane)carbamoyl]cholestérol (TC-cholestérol), du 3β[N-(N',N'-diméthylaminoéthane)-carbamoyl]cholestérol (DC-cholestérol), du 3β[N-(N'-monométhylaminoéthane)carbamoyl]cholestérol (MC-cholestérol), du 3β[N-(aminoéthane)carbamoyl]-cholestérol (AC-cholestérol), de la cholestéryloxypropane-1-amine (COPA), du N-(N'-aminoéthane)carbamoylpropanoïque tocophérol (AC-tocophérol), et du N-(N'-méthylaminoéthane) carbamoylpropanoïque tocophérol (MC-tocophérol).

6. Composition selon la revendication 1, dans laquelle le rapport des quantités de charges électriques entre le lipide cationique (N) et le médicament anionique (P) (N/P) est de 0,1 à 128.

7. Composition selon la revendication 1, dans laquelle le rapport entre le poids du complexe du médicament anionique et du lipide cationique (a) et le poids du copolymère séquencé amphiphile (b) (a/b x 100) est de 0,001 à 100 % en poids.

8. Composition selon la revendication 1, dans laquelle le lipide fusiogène est au moins l'un sélectionné dans le groupe constitué d'un phospholipide, d'un cholestérol, et d'un tocophérol.

9. Composition selon la revendication 8, dans laquelle le phospholipide est un ou plusieurs sélectionnés dans le groupe constitué d'une phosphatidyléthanolamine (PE), d'une phosphatidylcholine (PC), et d'un acide phosphatidique.

10. Composition de la revendication 8, dans laquelle le lipide fusiogène est un ou plusieurs sélectionnés dans le groupe constitué de la dilauroyl phosphatidyléthanolamine, de la dimyristoyl phosphatidyléthanolamine, de la dipalmitoyl phosphatidyléthanolamine, de la distéaroyl phosphatidyléthanolamine, de la dioléoyl phosphatidyléthanolamine, de la dilinoléoyl phosphatidyléthanolamine, de la 1-palmitoyl-2-oléoyl phosphatidyléthanolamine, de la 1,2-diphytanoyl-3-sn-phosphatidyléthanolamine, de la dilauroyl phosphatidylcholine, de la dimyristoyl phosphatidylcholine, de la dipalmitoyl phosphatidylcholine, de la distéaroyl phosphatidylcholine, de la dioléoyl phosphatidylcholine, de la dilinoléoyl phosphatidylcholine, de la 1-palmitoyl-2-oléoyl phosphatidylcholine, de la 1,2-diphytanoyl-3-sn-phosphatidylcholine, de l'acide dilauroyl phosphatidique, de l'acide dimyristoyl phosphatidique, de l'acide dipalmitoyl phosphatidique, de l'acide distéaroyl phosphatidique, de l'acide dioléoyl phosphatidique, de l'acide dilinoléoyl phosphatidique, de l'acide 1-palmitoyl-2-oléoyl phosphatidique, de l'acide 1,2-diphytanoyl-3-sn-phosphatidique, du cholestérol, et du tocophérol.

11. Procédé de préparation d'une composition pour délivrer un médicament anionique consistant en un médicament anionique, un lipide cationique, un copolymère séquencé amphiphile, facultativement au moins un lipide fusiogène, et une solution aqueuse ou un agent assistant pour une lyophilisation, lequel procédé comprend :
(a) la dissolution du médicament anionique et du lipide cationique dans un solvant organique miscible à l'eau ou un solvant mixte d'une solution aqueuse et d'un solvant organique, pour séparer les phases ;
(b) la séparation de la phase de solvant organique de (a) ;
(c) le mélange de la phase de solvant organique de (b) avec le copolymère séquencé amphiphile et l'élimination du solvant organique ; et
(d) l'ajout d'une solution aqueuse au mélange duquel le solvant organique est éliminé, pour former une micelle,
(e) facultativement l'ajout d'un agent assistant pour une lyophilisation pour lyophiliser, pour effectuer une lyophilisation,
dans lequel le médicament anionique forme un complexe avec le lipide cationique, et le complexe est piégé dans la structure de micelle polymère de type noyau-coque du copolymère séquencé amphiphile d'un copolymère di-séquencé de type A-B comprenant une séquence A hydrophile formant une coque et ayant un poids moléculaire moyen en nombre allant de 200 à 50 000 Dalton et une séquence B hydrophobe formant un noyau et ayant un poids moléculaire moyen en nombre de 50 à 50 000 Dalton dans une solution aqueuse, et dans lequel la teneur du copolymère séquencé amphiphile est de 40 à 99,98 % en poids sur la base du poids sec total de la composition, et un tocophérol, un cholestérol, ou un acide gras en C10 à C24 est facultativement chimiquement conjugué à un groupe hydroxyle de l'extrémité de la séquence hydrophobe,
dans lequel la séquence A hydrophile est un polyalkylèneglycol, et la séquence B hydrophobe est un ou plusieurs sélectionnés dans le groupe constitué d'un polyester, d'un polyanhydride, d'un polyacide aminé, d'un polyorthoester, et d'une polyphosphazine.

12. Procédé de préparation d'une composition pour délivrer un médicament anionique consistant en un médicament anionique, un lipide cationique, un copolymère séquencé amphiphile, et facultativement au moins un lipide fusiogène, et une solution aqueuse ou un agent assistant pour une lyophilisation, lequel procédé comprend :
(a') la dissolution du médicament anionique, du lipide cationique et du copolymère séquencé amphiphile dans un solvant organique miscible à l'eau ou un solvant mixte d'une solution aqueuse et d'un solvant organique ;
(b') l'élimination de la phase de solvant organique de (a') ; et
(c') l'ajout d'une solution aqueuse au mélange de (b') duquel le solvant organique est éliminé afin de former une micelle,
(d') facultativement l'ajout d'un agent assistant pour une lyophilisation pour lyophiliser, pour effectuer une lyophilisation,
dans lequel le médicament anionique forme un complexe avec le lipide cationique, et le complexe est piégé dans la structure de micelle polymère de type noyau-coque du copolymère séquencé amphiphile d'un copolymère di-séquencé de type A-B comprenant une séquence A hydrophile formant une coque et ayant un poids moléculaire moyen en nombre allant de 200 à 50 000 Dalton et une séquence B hydrophobe formant un noyau et ayant un poids moléculaire moyen en nombre de 50 à 50 000 Dalton dans une solution aqueuse, et dans lequel la teneur du copolymère séquencé amphiphile est de 40 à 99,98 % en poids sur la base du poids sec total de la composition, et un tocophérol, un cholestérol, ou un acide gras en C10 à C24 est facultativement chimiquement conjugué à un groupe hydroxyle de l'extrémité de la séquence hydrophobe,
dans lequel la séquence A hydrophile est un polyalkylèneglycol, et la séquence B hydrophobe est un ou plusieurs sélectionnés dans le groupe constitué d'un polyester, d'un polyanhydride, d'un polyacide aminé, d'un polyorthoester, et d'une polyphosphazine.

13. Procédé selon la revendication 11 ou la revendication 12, dans lequel le lipide fusiogène est ajouté dans l'étape (c) ou (a').

14. Procédé selon la revendication 11 ou la revendication 12, dans lequel le médicament anionique est un matériau d'acide nucléique.

15. Procédé selon la revendication 11 ou la revendication 12, dans lequel le lipide cationique est un ou plusieurs sélectionnés dans le groupe constitué du chlorure de N,N-dioléyl-N,N-diméthylammonium (DODAC), du bromure de N,N-distéaryl-N,N-diméthylammonium (DDAB), du chlorure de N-(1-(2,3-dioléoyloxy)propyl-N,N,N-triméthyl-ammonium (DOTAP), de la N,N-diméthyl-(2,3-dioléoyloxy)-propylamine (DODMA), du 1,2-diacyl-3-triméthylammonium-propane (TAP), du 1,2-diacyl-3-diméthylammonium-propane (DAP), du 3β-[N-(N',N',N'-triméthylaminoéthane)-carbamoyl]cholestérol (TC-cholestérol), du 3β[N-(N',N'-diméthylaminoéthane)carbamoyl]cholestérol (DC-cholestérol), du 3β[N-(N'-monométhylaminoéthane)-carbamoyl]cholestérol (MC-cholestérol), du 3β[N-(amino-éthane)carbamoyl]cholestérol (AC-cholestérol), de la cholestéryloxypropane-1-amine (COPA), du N-(N'-amino-éthane)carbamoylpropanoïque tocophérol (AC-tocophérol), et du N-(N'-méthylaminoéthane)carbamoylpropanoïque tocophérol (MC-tocophérol).

16. Procédé selon la revendication 11 ou la revendication 12, dans lequel le rapport de la quantité de charges électriques entre le lipide cationique (N) et le médicament anionique (P) (N/P) est de 0,1 à 128.

17. Procédé selon la revendication 11 ou la revendication 12, dans lequel le rapport entre le poids du complexe du médicament anionique et du lipide cationique (a) et le poids du copolymère séquencé amphiphile (b) (a/b x 100) est de 0,001 à 100 % en poids.


### <Sequence Listing>

<110> SAMYANG CORPORATION
<120> Pharmaceutical Composition Containing Anionic Drug and Preparation Method of the Same
<130> OPP-2009-3329-KR
<150> KR 10-2008-0134459
   <151> 2008-12-26
<150> KR 10-2009-0130794
   <151> 2009-12-24
<160> 4
<170> KopatentIn 1.71
<210> 1
   <211> 19
   <212> RNA
   <213> Artificial Sequence
<220>
   <223> GFP siRNA sense strand
<400> 1
   gcaagcugac ccugaaguu 19
<210> 2
   <211> 19
   <212> RNA
   <213> Artificial Sequence
<220>
   <223> GFP siRNA antisense strand
<400> 2
   aacuucaggg ucagcuugc 19
<210> 3
   <211> 19
   <212> RNA
   <213> Artificial Sequence
<220>
   <223> VEGF siRNA sence strand
<400> 3
   ggaguacccu gaugagauc 19
<210> 4
   <211> 19
   <212> RNA
   <213> Artificial Sequence
<220>
   <223> VEGF siRNA antisense strand
<400> 4
   gaucucauca ggguacucc 19